# EUROPEAN PATENT SPECIFICATION

(11) **EP 1 722 859 B1**
(45) Date of publication and mention of the grant of the patent: **08.09.2010**
(21) Application number: 04802165.3
(22) Date of filing: 15.12.2004
(51) Int. Cl.: A61Q 5/00, A61Q 17/02, A61Q 17/04, A61Q 19/00, A61Q 19/04, A61K 8/58

(54) **A BASE COMPOSITION FOR PREPARING MULTI-FUNCTIONAL FORMULATIONS FOR CARE AND PROTECTION OF THE SKIN AND HAIR**
BASISZUSAMMENSETZUNG ZUR HERSTELLUNG VON MULTIFUNKTIONALEN FORMULIERUNGEN FÜR DIE PFLEGE UND DEN SCHUTZ VON HAUT UND HAAREN
COMPOSITION DE BASE POUR LA PREPARATION DE FORMULATIONS MULTIFONCTIONNELLES POUR LE SOIN ET LA PROTECTION DE LA PEAU ET DES CHEVEUX

(30) Priority: 15.12.2003 BR 0308051
(43) Date of publication of application: 22.11.2006
(73) Proprietor: Natura Cosméticos S.A., CEP-04752-900 Sao Paulo, SP (BR)
(72) Inventor: FUSCELLI PYTEL, Rodrigo, CEP-07750-000 Sao Paulo, SP (BR); PERASSINOTO, Nelson, Luis, CEP-13031-440 Campinas, Sao Paulo, SP (BR); VILLA NOVA SILVA, Luciana, CEP-05688-000 Sao Paulo, SP (BR); DOS SANTOS SILVA, Ana, Luisa, CEP-13026-280 Campinas, Sao Paulo, SP (BR); STORCK NUNES, Almir, CEP-05056-000 Lapa, Sao Paulo, SP (BR)
(74) Representative: Senior, Janet
(86) International application number: PCT/BR2004/000244
(87) International publication number: WO 2005/060923

(56) References cited:
- WO-A-01/17496
- US-A1- 2003 124 078
- US-A1- 2004 161 395
- US-B1- 6 387 382

## Description

The present invention relates to a cosmetic composition comprising a filmogenic system and an emulsifying system. This composition imparts to the skin high resistance to water and to sweat and provides prolonged hydration. Further, it imparts to the hair long retention of curls, as well as significant increase on brightness and strengthening of the hair.

### Description of the Prior Art

In many western cultures having a tanned body is synonym of status and beauty, and voluntary exposure to the sunlight, in general associated to physical activities, beach or swimming pool, is a quite common practice. However, although the sunlight is fundamental to health, there are numberless deleterious effects on the skin and hair.

With a view to make exposure to the sunlight safer, a number of preparations to be used topically and on hair, known as sunscreens, have been developed. These preparations have, in their formulations, molecules that are generically classified as sun filters, which act by basically two different mechanisms: absorption or reflection of diverse wave lengths present in the sunlight, as those found in the spectral ranges known as UVA and UVB.

In order to have the sunscreens with an adequate performance in real conditions of use, that is to say, associated to physical activities and baths in swimming pools or in the sea, their capability of remaining on the skin or hair should be increased. This increase can be obtained by the presence of polymeric macromolecules with high substantivity and by the filmogenic action or else by the selection of adequate emulsifiers.

Polyvinyl pyrrolidones, polyvinyl alcohols and some classes of silicones constitute the main known categories of substances that exhibit filmogenic action.

However, in general, the utilization of these substances guarantees only a period of resistance to water and varies from 40 to 80 minutes, proven through known international protocols, and come from the obtaining of preparations that, in general, exhibit the following characteristics:
1 - undesirable levels of oiliness and stickiness;
2 - low spreading degree;
3 - difficult absorption of the composition by the skin;
4 - possibility of raising the degree of oiliness of the skin;
5 - occurrence of possible cases of comedogenicity by occlusive action on the skin;
6 - incapability of forming a homogeneous film on the skin, resulting in an unsatisfactory performance with respect to resistance to water and to sweat, and resulting in "rolling" (areas of accumulation of the product, because it does not spread adequately);
7 - they do not have adequate resistance to moisture, and consequently do not provide a satisfactory retention of curls;
8 - due to the constitution of the known compositions, the polymer comprised therein, which is deposited onto the hair, soon gets loose from it, thus causing the "flake effect" (scaling of the polymer particles that were covering the hair);
9 - they do not provide brightness, besides not increasing the resistance of the hair.

Some patent documents describe silicone compositions, as for example:

Document BR 0013874 describes a cosmetic composition for care of the skin without oil, which comprises emollients such as dimethicone, dimethicone copolyol and trimethylsiloxysilicate. In addition to these components, said composition contains water, moistener and a keratolytic agent. The function of this composition is to provide softening to the skin due to the presence of emollients and also to retain moisture in order to prevent loss thereof.

The presence of the keratolytic agent in the described cosmetic composition promotes the weakening of the hair.

Document US 5,478,552 describes a liquid cosmetic material comprising a volatile silicone type and trimethylsiloxysilicic acid. It further comprises silicic anhydride, fatty acid ester and mineral clay. This cosmetic exhibits resistance to water, among other properties.

Mineral clay renders the application of the composition onto the hair and the skin of men very difficult, which naturally has much hair. The presence of silicic anhydride and mineral clay promotes astringent actions, which impairs the action of silicones and of fatty acid ester.

Document US 6,387,382 describes preparations for the skin with multiple functionalities in the form of oil-in-water emulsions. Theses preparations comprise a siloxane polymer such ad dimethicone, a cyclic silicone and emollient. These preparations are resistant to water by virtue of a protecting barrier formed.

The combination dimethicone, a cyclic silicone and emollients provide an inadequate time of resistance to water in comparison with which is expected by the users.

### Summary of the Invention

The invention relates to a cosmetic composition, particularly indicate for use in tanning formulations, sunscreens or cosmetic preparations for use on the hair, which comprise a filmogenic system comprising at least cyclopentasiloxane, dimethiconol, dimethicone, trimethylsiloxysilicate and stearyl dimethicone, and an emulsifying system comprising at least PEG-8 dimethicone. This cosmetic composition may further contain various components known in the cosmetology area, such as moisteners, conditioning agents, photoprotecting agents, skin and hair toning agent, fragrance, among others.

### Brief Description of the Figures

The present invention will now be described in greater detail with reference to the figures that illustrate the tests of performance of the present invention.
- Figure 1 is a graph representing numerically the appraisal of the loss of curl shaping (LCS) over time;
- Figure 2 is a graph representing numerically the appraisal of the increase in tension in maximum load (TML) on the treated hair in comparison with untreated hair (control); and
- Figure 3 is a graph representing numerically the appraisal of the energy at the rupture point on treated hair in comparison with untreated hair (control).

### Detailed Description of the Invention

The present invention describes a cosmetic composition comprising a filmogenic system that comprises at least cyclopentasiloxane, dimethiconol, dimethicone, trimethylsiloxysilicate and stearyl dimethicone, and an emulsifying system comprising at least PEG-8 dimethicone. In addition to the cited components, it may further comprise photoprotecting agents, skin and hair toning agent and, optionally, other agents that have specific functions required for each composition necessary for each situation, such as, chelating agents, thickening agents, pH-adjusting agents, preservative agents, moistening agents, conditioning agents, emollients, optional silicones, optional filmogenic agents, oiliness absorbing agents, skin and hair toning agents, optical diffusion agents, skin tensioning agents, sunscreens and filters against ultraviolet rays or active ones, as bacteriostatic, bacterial or antimicrobial, anti-radical, anti-aging, anti-inflammatory, among others. This composition may be called a cosmetic base, since it may receive a number of optional components in its constitution.

This cosmetic composition, when applied onto the skin, provides mainly a high resistance to water and to sweat, as well as prolonged hydration. When applied to the hair, its filmogenic action on the hairs provides high retention of curls, a significant increase in brightness and strengthening of the individual hairs.

Further, according to the optional components added to the composition, the latter can provide softness, smoothness, protection against the sunlight, with good spreading and without increasing the oiliness.

The cosmetic composition of the present invention comprises the association of specific filmogenic agents and emulsifying agents to obtain a variety of formulations with diverse functionalities for the care of the skin and the hair. Each of the components, either alone or in combination, of this base has a quite advantageous purpose for the care of the skin or hair, namely:
- the combination of dimethicone and trimethylsiloxysilicate provides repellence to water and also imparts to the product containing them good spreading and does not cause occlusive effects;
- with the present invention, PEG-8 dimethicone acts as an agent that promotes homogeneity of the emulsions and further imparts good conditioning to the skin;
- with the present cosmetic composition, stearyl dimethicone has the function of providing hydration to the skin due to the fact of forming a smooth film on the surface of the skin;
- the combination of cyclopentasiloxane and dimethiconol acts on the skin as a film forming system, further providing smoothness and softness;
- the combination of dimethicone, trimethylsiloxysilicate and PEG-8 dimethicone, at the concentrations indicated in this invention, which will be described later, imparts to the formulations in which it is contained a period of 80 minute of resistance to water;
- the combination of dimethicone, trimethylsiloxysilicate, cyclopentasiloxane, dimethiconol, stearyl dimethicone, PEG-8 dimethicone provides high resistance to water and to sweat, as well as prolonged hydration, when applied to the skin. It also provides a long time of retention of curls, a significant increase in brightness and strengthening of the individual hairs, when applied thereto.

Although described as being particularly useful for formulations for sunscreens, the cosmetic composition of the present invention has a variety of advantages and desired characteristics in a cosmetic product for the skin or hair, some of which are listed below.
1 - it is stable for a period of at least two years;
2 - it has an adequate texture during application, non-sticky, non-oily;
3 - it is easy to spread;
4 - it exhibits an ideal time relative to absorption and drying of the cosmetic composition on the skin;
5 - it does not cause oiliness on the skin after application;
6 - it has high capacity of maintaining hydration of the skin;
7 - it does present comedogenicity;
8 - it does not present phototoxicity;
9 - it does not present allergicity;
10 - it has resistance to water, even after a period of immersion of 360 minutes;
11 - it has resistance to sweat, even after a period of forced sudoresis of 120 minutes;
12 - it does not cause any type of adverse reaction or lesion, on the skin or in the eye, whether in normal conditions of use or in conditions of forced sudoresis;
13 - it is compatible with agents that protect against the sunlight (sunscreens) at concentrations that guarantee sunscreen protection factors (SPF) between 2 and 100, preferably from 4 to 60;
14 - it is compatible with agents that protect against the sunlight (sunscreens) at concentrations that guarantee blocking of over 50% of the UVA rays, preferably over 80% of these rays;
15 - it is associable with emollients so as to provide a degree of hydration of the skin significantly higher than that measured for the control area for more than 24 hours after a single application, preferably for more than 8 hours;
16 - it guarantees a significant increase in brightness of the hair;
17 - it provides retention of curls significantly longer than the retention measured in control areas (without application of product), for more than 8 hours, after a single application in conditions of high relative moisture;
18 - it promotes an increase by 35% in the tension verified in the maximum load applied to the hair and 53% in the energy necessary to break the hair thread, which results in the strengthening of the individual hairs.

The cosmetic composition of the present invention can serve as a base for any other composition that may contain some type active principle for the skin other than designed only for protection against the sunlight. A few examples of products that can be prepared from the cosmetic composition of the present invention are:
- sunscreens for adults or children, either for concomitant use in practicing sports or not;
- body or face hydrating products;
- products against spots on the body or face;
- body or face firming products;
- self-tanning products;
- insect repellent products;
- body or face hydrating agents that enlighten the skin;
- pharmaceutical preparations of topical application, that require protection against solar radiation at the application place;
- cosmetic preparations for the body or face intended for children;
- localized-action cosmetic preparations, specific for periocular region, around the lips, on the lips, antispots, against shadows under the eye, among others;
- non-rinsable hair conditioners;
- sunscreens for hair;
- pharmaceutical preparations having action on the hair that require, at the place of application, high resistance to water, as for example pharmaceutical shampoos;
- rinsable or non-rinsable hair preparations that aim at an increase in brightness and /or a long time of retention of curls and/or the strengthening of the individual hairs.

### Filmogenic System

The filmogenic system is constituted by at least dimethicone, trimethylsiloxysilicate, stearyl dimethicone, cyclopentasiloxane and dimethiconol. The amount of this system in the composition ranges from 0.3% to 92% by weight, preferably from 0.5% to 50.0% by weight, based on the total weight of the cosmetic composition. Silicone agents might be added to this system, which will be described later.

By preference, combinations of dimethicone and trimethylsiloxysilicate are used, which have the following properties (measured at a temperature of 25° C):
- refraction index: 1.408 to 1.410
- viscosity: 400 - 1020 cP.

Further, preferably combinations of cyclopentasiloxane and dimethiconol which have the following properties are used (measured at a temperature of 25°C):
- viscosity: 4500 - 8000 cP
- relative density: 0.945 - 0.965 g/cm³.

### Emulsifying System

The emulsifying system is constituted by at least PEG-8 dimethicone. The amount of this system in the composition ranges from 0.1 % to 28.0% by weight, preferably from 0.3% to 12.0% by weight, based on the total weight of the composition. Like the preceding system, this system may further receive optional emulsifying agents, which will be defined later.

### Constitution of the Cosmetic Composition

In the following table, the preferred proportions of the agents that constitute the filmogenic and emulsifying systems described above are given. These systems combined, in the amounts corresponding to the proportions defined in the following table, in the form of products for topical use or for use on the hair, enable to obtain preparations that exhibit significant improvements in comparison with the known preparations of topical use that, which provides resistance to water and sweat. Further, these improvements are noted in the sensorial aspects related with the safety and effectiveness of use of this composition on the skin:

| Component | Preferred range of proportions (parts by weight) |
|---|---|
| Dimethicone and trimethylsiloxysilicate | 8 - 12 |
| Stearyl dimethicone | 7 - 11 |
| PEG-8 dimethicone | 4 - 7 |
| Cyclopentasiloxane and dimethiconol | 3 - 7 |

### Silicone Agent

The types of silicone agents that, in the present invention, act as filmogenic agents exhibit emollient, solvent and skin conditioning properties. A few examples of silicone that may be added to the cosmetic compositions of the present invention as optional filmogenic agents are volatile and nonvolatile silicone oils such as, for example, cyclomethicone, alkyldimethicones, dimethicone-copolyols, phenyl trimethicones, caprilyl trimethicones, aminofunctional silicones, silicones modified with phenyl, phenyl trimethicones, alkyl modified silicones; dimethyl and diethyl polysiloxane, mixed C₁-C₃₀ alkyl polysiloxane, dimethyl siloxanes, polymethylsiloxane, alphamethyl-omega-metoxypropymethylsiloxane, polyoxydimethylsilylene, polydimethyl silicone and combinations thereof or silicone elastomer such as cyclomethicone and dimethicone crosspolymer, vinyl dimethicone and dimethicone crosspolymer, dimethicone and dimethicone crosspolymer and cyclopentasiloxane and dimethicone crosspolymer.

Further, as optional filmogenic agents, some polymers such as polyvinyl pyrrolidones and polyvinyl alcohols can be added.

In the preferred embodiments of the present invention, preferably cyclomethicone or dimethicone copolyol or a combination thereof is used as an optional filmogenic agent.

### Optional Emulsifying Agent

As optional emulsifying agents, anionic emulsifiers, zwiterionic emulsifiers, non-ionic emulsifiers and polymeric emulsifiers can be used.

A few examples of emulsifying agents may be added to those which make part of the described emulsifying system, such as soybean lecitin, nonyl phenol ethoxylate, sodium or potassium alkyl phosphates, dietanolamine cetyl phosphate, dimethicone copolymers such as modified polyoxyalkylene dimethylpolysiloxanes, methyl glucose, propylglyceryl glucose, ethoxylated glycols, non-ethoxylated glycols, ethoxylated fatty acids, non-ethoxylated fatty acids, ethoxylated sucrose, non-ethoxylated sucrose, ethoxylated pentaeritritol, non-ethoxylated pentaeritritol, ethoxylated alkyl glycosides, non-ethoxylated alkyl glycosides, ethoxylated alkyl polyglycosides, alkyl phosphates, propylene glycol, and derivatives thereof and mixtures thereof.

Other alternatives of compounds that can act as emulsifying agents are esters such as potassium cetylphosphate, glyceryl stearate, ethoxylated sorbitan esters, non-ethoxylated sorbitan esters, such as sorbitan stearate, ethoxylated sorbitan stearate, polysorbate 80, polysorbate 65, polysorbate 60, polysorbate 40, polysorbate 20, polyquaternium 37, glycol stearate, PEG-2 stearate, PEG-10 stearate, PEG-30 stearate, PEG-40 stearate, PEG-100 stearate, PEG-150 stearate, PEG-1000 stearate, sorbitan palmitate, propylene glycol stearate, propylene glycol stearate PEG-25, sorbitan oleate, methyl glycose sesquistearate, among others.

Further, fatty alcohols such as cetyl alcohol, stearyl alcohol, cetearyl alcohol can be used. Propoxylated and/or ethoxylated fatty alcohols, among others.

Moreover, there are other options as synthetic polymers, which, besides having the function of a thickening agent, also have the function of a polymeric emulsifying agent, which are formed by carboxyvinyl polymers and copolymers, acrylates, methacrylates, alkyl acrylates, acrylate / C₁₀-C₃₀ alkyl acrylate crosspolymer, acrylamides, taurates and/or combinations thereof, in addition of ethylene oxide and propylene oxide copolymers.

Further, sterols such as ethoxylated or non-ethoxylated animal sterols, ethoxylated vegetable sterols, lanolin and derivatives, for example, lanolin alcohol, cholesterol, soybean sterol, hydrogenated lanolin, hydroxylated lanolin and derivatives thereof should be considered.

Preferably, non-ionic emulsifiers, polymeric emulsifiers and alkyl phosphates should be used, more preferably potassium cetyl phosphate at a concentration ranging from 0.1 % to 15.0% by weight, preferably from 0.5 to 3.0% by weight, based on the total weight of the composition of the invention.

The choice of an optional emulsifying agent should be made with extreme care, since it may significantly after the effectiveness and safety of the cosmetic composition.

In addition to the above-mentioned components, the compositions of the invention may further comprise compounds conventionally used in cosmetic compositions of this time, which will be detailed hereinafter.

### Chelating Agent

The use of a sequestering agent or chelating agent is due to the fact it has the property of sequestering ions from the solution; they are capable of sequestering calcium and magnesium atoms, but preferably they exhibit selectivity for linking to ions such as iron, manganese and copper. Their function is to control a possible oxidation action that may occur and further to promote stability in stocking the cosmetic compositions of the present invention.

A few examples of chelating agents that may be added to the compositions of the present invention include etiodronic acid, nitrile acetic acid, polyaminocarboxylic acid such as ethylenediaminotetracetic acid (EDTA) and salts thereof, pentaacetic ethylenetriamine acid, ethylenediamine disuccinic acid, amono phosphanates, nitrile acetates, hydroxyethylethylene triamines, organic phosphates such as sodium hexamethaphosphate, sodium tripolyphosphate, sodium gluconate, stilbene, ethylene diamine diglutaric acid, EGTA and salts thereof, solvates, isomers and derivative.

In the preferred embodiments of the present invention, preferably disodic EDTA is used to act as a chelating agent in an amount ranging preferably from 0.05% to 0.20% by weight, preferably about 0.10% by weight, based on the total weight of the composition of the invention.

### Thickening Agent

The function of the thickening agent in cosmetic compositions is to maintain in suspension other components present therein, besides providing consistency to the compositions.

A few examples of thickening agents that may by used in the present invention are: natural polymers, carbomer, or preferably types of gum such as acacia gum, agar, tara gum, gum arabic, algin,alginic acid and derivatives such as ammonium or sodium alginate, amilopectin, calcium alginate, gelatin, gelan gum, guar gum, sodium hyalurionate, guar hydroxypropyl, Karaya gum, carob bean oil, natto gum, potassium alginate, potassium carrageenin, sclerotium gum, sodium carrageenin, dragantum gum, xanthan gum and mixtures thereof.

Xanthan gum exhibits excellent thickening properties, in addition to adequate suspending and pseudoplastic properties. These characteristics allow the cream to spread easily over the skin. In this regard, xanthan gum is the preferred gum for addition to the present invention.

Further, polysaccharides may be used, as thickening agents, in the compositions of the present invention. These polysaccharides have a structure with repeated sugar units such as, for example, a carbohydrate. Among various examples, the following may be cited: cellulose and derivatives thereof such as hydroxyethyl cellulose, carboxymethyl hydroxyethyl ce-Ilulose, ethyl cellulose, hydroxyethyl ethyl cellulose, hydroxypropyl cellulose, hydroxypropyl methyl cellulose, methyl hydroxyethyl cellulose, microcrystaline cellulose, sodium sulfate cellulose, hydroxypropyl cellulose and mixtures thereof. Other examples of polysaccharides that may be added are celluloses with alkyl substituted as for example: stearyl, isostearyl, lauryl, miristil, cetyl, isocetyl, cocoyl, palmityl, oleyl, linoleyl and mixtures thereof, in addition to scleroglucanes and derivatives thereof.

Other alternatives are the synthetic polymers that also act as polymeric emulsifying agents formed by carboxyvinyl polymers and copolymers acrylates such as alkyl C₁₀₋₃₀ acrylate, methacrylates, alkyl acrylates, acrylamides, taurates, acrylate crosspolymers and/or combinations thereof.

In the preferred embodiments of the present invention, preferably a thickening system composed by carbomer and xanthan gum is used in an amount ranging from 0.01% to 5.00% by weight, preferably from 0.10% to about 1.00% by weight, based on the total weight of the cosmetic compositions.

### Ph Adjusting Agent

In order to obtain a final composition with neutral pH values or suitable for the skin, the following may be added to the composition of the present invention: inorganic hydroxides such as sodium hydroxide, calcium carbonate, citric acid, phosphoric acid, lactic acid, sodium citrate, succinic acid, potassium acetate, sodium chloride, amines such as tertiary amine, triethanolamine and mixtures thereof.

In the preferred embodiments of the present invention, triethanolamine is used as a pH adjusting agent in an amount ranging from 0.01% to 5.00% by weight, preferably 0.1% to 1.0% by weight, based on the total weight of the composition.

### Preserving Agent

A preserving agent, as its name indicates, provides preservation of the composition to which it is added, that is to say, it provides the composition with effective protection against attack by microbial agents, prolonging its useful life or shelf life.

There are a number of preserving agents suitable for cosmetic compositions, and all the agents that have this function may be added to the cosmetic composition of the present invention, either alone or in combination.

A few preferred examples of preserving agents for addition to the composition of the present invention are: sodium acetate, boric acid, sorbic acid, benzalkonium chloride, sodium metalbisulfite, potassium sorbate, sodium sulfite, calcium propionate, glutaraldehyde, cream of tartar, sodium benzoate, organic acids, lactic acid, benzoic acid, hydroxymethylglycinates, parabens such as methylparaben and propylparaben, phytic acid, PEG-5 cocoate, PEG-8 dicocoate, iodopropinyl PEG-4 buthyl carbamate, phenoxyetanol, formaldehyde solution, pentanediol, phenol alcohol, benzyl alcohol, iodo alkyl carbamates, imidazoleidinyl, 1,3-bis(hydroxymethyl)-5,5-dimethyl-2,3-imidazolidione, methylchloroisothiazolidione, methylisothazolinone and derivatives thereof and/or combinations thereof.

In the preferred embodiments of the present invention, phenoxiethanol or 3-iodo-propynylbutyl carbamate or a combination thereof is used as a preserving agent in an amount ranging from 0.01% to 2.00% by weight, preferably from 0.10% to 1.00% by weight, based on the total weight of the composition.

### Moistening Agent

The function of the moistening agent in the cosmetic composition of the present invention is to promote retention of water on the user's skin, that is to say, to provide the skin with water and prevent loss of water from it. The moistening agent further helps in increasing the effectiveness of the emollient, reduces scaling of the skin and improves the sensorial capacity of the skin.

A few examples of moistening agents that may be added to the cosmetic compositions of the present invention are: PEG-4 dilaurate, polyhydroxyl alcohols, alkylene polyols and derivatives thereof, clycerol, ethoxylated glycerol, propoxylated glycerol, sorbitol, hyroxypropyl sorbitol, among others, glycerin, lactic acid and lactate salts, diols and C₃-C₆ triols, *Aloe vera* extract, butylenes glycol, sugars and amides and derivatives thereof as for example alkoxylated glucose, hyaluronic acid, glycolic acid, lactic acid, glycolic acid and salicylic acid, panteol, urea, ethoxylated nonyl phenol, natural oils such as pine oil, oils and waxes and mixtures thereof.

In the preferred embodiments of the present invention, at least one glycol or *Aloe vera* extract, or a combination thereof is used as a moistening agent in an amount ranging from 0.01 % to 30.00% percent, preferably from 0.05% to 15.00% by weight, based on the total weight of the composition.

### Emollient

The function of the emollients in cosmetic compositions is to add and replace lipids and natural oils to the skin. They also act as solubilizers of sunscreens.

As emollients for addition to the composition of the present invention, conventional lipids may be added such as, for example, oils, lipids and other water-insoluble components and polar lipids that are modified lipids so as to increase its water solubility by esterifying a lipid in a hydrophilic unit such as for example hydroxyl groups, carbonyl groups, among others. A few compounds that may be used as emollients are natural oils such as essential oils and derived from plants, esters, silicone oils, polyunsaturated fatty acids, lanolin and derivatives. A few natural oils that may be used are derived from damask seed, sesame seed, soybean, peanut, coco-nut, olive, cocoa butter, almond, avocado, carnauba, cotton seed, rice flour, peach stone, mango stone, jojoba, macadamia nut, coffee beans, grape seeds, pumpkin seeds, among others and mixtures thereof.

A few ethers and esters may also be used in the function of emo-Ilients, such as, for example, C₈-C₃₀ alkyl esters of C₈-C₃₀ carboxylic acids, C₁-C₆ diol monoesters and C₈-C₃₀ carboxylic acid diesters, C₁₀-C₂₀ alcohol monoester sucrose and combinations thereof. Examples of these compounds are dicaprylic ether, cetyl lactate, isopropyl palmitate, dicaprylyl carbonate, C₁₂-C₁₅ alkyl benzoate, isopropyl miristate, isopropyl isononate, sucrose palmitate, sucrose oleate, isostearyl lactate, glyceryl behenate, triglycerol-4 isostearate, lauryl pyrrolidone carboxylic acid, pantenyl triacetate and combinations thereof.

Also, other fatty alcohols may be used as mono-, di- or triclyceride esters that have a lipophilic nature, such as dicraprylylic ether, in addition to synthetic and natural hydrocarbons, organic carbonates such as dicaprylyl carbonate, a few types of silicones such as cyclomethicone and mixtures thereof.

In addition, different natural compounds may be used as emollients, such as microcrystalline wax, carnauba wax, Shea butter, bee wax, ozokerite wax, among others.

In the preferred embodiments of the present invention, C₁₂-C₁₅ alkyl benzoate, dicaprylyl carbonate or a combination thereof is used as an emollient agent in an amount ranging from 0.01% to 2.00% by weight, preferably from 0.10% to 1.00% by weight, based on the total weight of the composition.

### Oiliness Absorbing Agent

As an agent for modifying the sensorial capacity, that is to say, to promote adsorption of the oiliness, various compounds may be added to the composition of the present invention, such as: polyethylene, polymethyl acrylates such as polymethylmethacrylate, acrylate polymers, salicylicate ester, niacinamide, aluminum silicate, magnesium silicate, calcium silicate, magnesium carbonate, calcium carbonate, magnesium oxide, magnesium hydroxide, titanium dioxide, zinc laurate, zinc miristate, polyacrylamide, polysaccharides, modified polysaccharides, cellulose, microcrystalline cellulose maize starch, glyceryl starch, maltodextrin, borates, nitrates such as boron nitrate, silicas such as colloidal silica, hydrated silica, talc, Nylon such as Nylon 12, mica such as titanated mica, chlorides such as bismuth chloride, modified starch such as octenylsuccinate aluminum starch and derivatives thereof, bentonites, besides mixtures thereof.

In the preferred embodiments of the present invention, Nylon 12 or octenylsuccinate aluminum starch or a combination thereof is used as a sensorial capacity modifying agent in an amount ranging from 0.1% to 40.0% by weight, preferably from 1.0% to 10.0% by weight, based on the total weight of the composition.

### Skin-toning Agent

Optionally, skin-toning agents may be added for imparting this characteristic to the cosmetic composition of the present invention. The types of skin-toning agents suitable for the composition in question are those that react with the proteins of the skin or those that only dye it, such as those constituted by ultrafine dispersions of inorganic pigments and oxide nanopigments or still those constituted by vegetable substances.

A few examples of skin-toning agents indicated for the present composition are: erythrulose and dihydroxyacetone (DHA), iron oxide, ditanium dioxide, zinc oxide, those mono or poly carbonyl compounds such as isatin, aloxane, ninidrin, glyceraldehydes, mesotartaric aldehyde, glutaraldehyde, derivatives of pyrazolin-4,5-dione or of 4,4-dihydroxypyrazolin-5-one, methylglyoxal, 2,3-dihydroxysuccinic dialdehyde, 2-amino-3-hydroxysuccinic dialdehyde and 2-benzylamino-3-hydroxysuccinic dialdehyde.

In the preferred embodiments of the present invention, Erythrulose or dihydroxyacetone (DHA) or a combination thereof is used as skin-toning agent in an amount ranging from 0.5% to 20.0% by weight, preferably from 1.5% to 6.0% by weight, based on the total weight of the composition.

### Sunscreens

In order to filter the sun rays, sunscreens may be added, which may be water-soluble or liposoluble

A few examples of sunscreens that absorb ultraviolet rays, which are indicated for addition to the cosmetic composition of the present invention are: benzilidene camphor and derivatives thereof, isophthalilidene camphor and derivatives thereof, cinnamic acid and esters thereof, salicylic acid and esters thereof, benzoic acid and esters thereof, p-aminobenzoic acid and derivatives thereof such as its esters, substituted hydroxybenzophenones, substituted dibenzoylmethane, benzotriazol and a few derivatives such as 2-arylbenzotriazol, 2-arylbenzimidazol, 2-arylbenzofuranes, 2-arylbenzoxazol, 2-arylindol, mono-phenylcyanoacrylates, diphenylcyanoacrylates, among other sunscreens against ultraviolet rays known from the prior art.

A few examples of sunscreens indicated for the present invention are organic compounds which often have low water-solubility such as triazine derivatives (for example, hydroxyphenyltriazine compounds or benzotriazol derivatives), some amides such as those that contain a vinyl group, cinnamic acid derivatives, sulfonated benzimidazoles, diphenylmalonitryls, oxalylamides, camphor derivatives, salicylic acid derivatives such as 2-ethylhexyl salicylates, homosalates and isopropyl salicylates, benzophenone derivatives such as benzophenone-2, benzophenone-3 and benzophenone-4, PABA such as 2-ethylhexyl 4-dimethylamino-benzoate and other sunscreens usually added to the compositions of products for protection against the sunlight.

In addition to the examples cited above, there are other preferred components for performing this function, namely: anillino N,N-trimethyl-4-(2-oxoborn-3-ilidenomethyl) methyl sulfate; 3,3'-(1,4-phenylenodimethylene) bis (7,7-dimethyl-2-oxo-bicyclo-(2.2.2) 1-heptylmetanosulfonic acid and derivatives thereof; 1-(4 terc-butylphenyl)3-(4-methoxyphenyl) propane-1, 3, dione; alpha-(2-oxoborn-3-ilidene) toluene-4-sulfonic acid and salts of potassium, sodium and triethanolamine; 2-cyano-3, 2-ethylexyl 3'-diphenylacrylate; 2-ethoxyethyl 4-methoxycynnamate; methyl anthraninate; Triethanolamine salicylate; 2, 2', 4, 4' tetrahydroxybenzophenone; 2-phenylbenzimidazol-5-sulfonic acid and its salts of potassium, sodium and triethanolamine; 2-ethylhexyl 4-methoxicynnamate; 2-hydroxy-4-methoxybenzophenone (oxybenzone); 2-hydroxy-4-methoxybenzophenone 5-sulfonic acid and its sodic salt (sulisobenzone and sodic sulisobenzone); aminobenzoic PABA acid; homomethyl salicylate; N-{(2 and 4) [(2 oxoborn-3-ilidone) methyl] benzyl} acrylamide polymer; titanium dioxide (with or without a lipophilic coating); ethyl N-ethoxy-4-aminobenzoate; 2-ethylhexyl 4-dimethylaminobenzoate; 2-ethylhexyl salicylate; isopentyl 4-methoxycinnamate; 3-(4'-methylbenzilide-no)-d-1 camphor; camphor 3-benzilidene; 2, 4, 6-trianilin-(p-carbo-2'ethyl-hexyl-l'-oxy)-1,3,5-triazine octyl; zinc ozide (with or without lipophilic coating); 2-(2H-benzotriazol-2-yl)-4-methyl-6-{2-methyl-3-(1,3,3,3-tetramethyl-1-((trimethylsilyl)oxy) disiloxanyl) propyl} phenol; benzoic acid; 4,4'-[[6-[[4-[[1,1-diomoethyl-ethyl] carbonyl] amino]-1,3-5-triazine-2,4-diyl] diimino] bis, bis (2 ethylhexyl); 2,2'-methylene-bis-6-(2H-benzotriazol-2-yl)-4-(tetramethyl butyl)-1,1,3,3-phenol; methylene bis-benzotriazolyl tetraethyl butyl phenol; monosodic salt of 2,2'-bis-(1,4-phenylene)-1H-benzimidazol-4,6 6 disulfonic acid; (1,3,5)-triazine-2,4-bis {[4-(2-ethyl-hexyloxy)-2-hydroxy]-phenyl]-6-(4-methoxyphenyl); bis-ethylexyloxyphenol methoxyphenyl triazine; methylene bis-benzotriazolyl tetramethylbutyl phenol; butyl methoxydibenzoyl methane, 2-ethylhexyl p-methoxycinnamate, methylene bis-benzotriaolyl tetramethylbutyl phenol, 4-butyl-4-methoxydimenziolmethane, benzophenone 3, bis-ethylhexyloxyphenol methoxyphenyl trazine, octhyl triazone, titanium dioxide, cinnamidopropyltrimonium chloride, dimethylpabamidopropyl laurdimonium tosylate and still other non-mentioned sunscreens.

In the preferred embodiments of the present invention, for products of topical application, the mixture of benzophenone-3, butyl methoxydibenzoylmethane, 2-ethylhexyl p-methoxycinnamate and bis-ethylhexyloxyphenol methoxyphenyl triazine is used as a sunscreen system, in an amount ranging from 0.10% to 50.0% by weight, preferably from 1.0% to 25.0% by weight, based on the total weight of the composition. On the other hand, for products intended to be used on the hair, the mixture of cinnamidopropyltrimonium chloride and dimethylpabamidopropyl laurdimonium tosylate is used as a sunscreen system in an amount ranging from 0.1% to 30.0% by weight, preferably from 0.5% to 15.0% by weight, based on the total weight of the composition.

Accordingly, in one embodiment of the present invention, there is provided a cosmetic composition as defined above, characterized by comprising a sunscreen system in an amount ranging from 0.1 % to 50.0% by weight. In another embodiment of the present invention, there is provided a cosmetic composition as defined above, characterized by comprising a sunscreen system in an amount ranging from 0.1% to 30.0% by weight.

In one embodiment of the present invention, there is provided a cosmetic composition as defined above, **characterized in that** the sunscreen system is composed of benzophenone-3, butyl methoxydibenzoylmethane, 2-ethylhexyl p-methoxycinnamate and bis-ethylhexyloxyphenol methoxyphenyl triazine. In an alternative embodiment of the present invention, there is provided a cosmetic composition as defined above, **characterized in that** the sunscreen system is composed of cinnamidopropyltrimonium chloride and dimethylpabamidopropyl laurdimonium tosylate.

### Antioxidant Agent

The antioxidant agents act to protect the topical composition and the skin against oxidation action.

Compounds that have antioxidant properties and may be added to the compositions of the present invention are: sulfites, ascorbates, amino acids (for example, glycine, histidine, tyrosine and triptofane), amidazoles, urocanic acid and derivatives thereof, peptides (for example, D, L-carnosine, D-canosine and L-carnosine), anserine, carotenoids, carotenes and derivatives thereof (for example, alpha-carotene and beta-carotene), licopene and derivatives thereof, chlorogenic acid and derivatives thereof, lipoic acid and derivatives thereof, dihydrolipolc acid, aurothloglycose, propylthiouracyl, thioredoxyne, glutathione, cisteine, cistine, cistamine, dilauryl thiodipropionate, diestearyl thiodipropionate, thiodipropyonic acid, salts of sulfoximine compounds, palmitic acid, phytic acid, citric acid, lactic acid, malic acid, pholic acid, vitamin C, tocopherols, vitamin A, vitamin E, vitamin E acetate, lipophilic substances such as butyl hydroxytoluene (BHT), butyl hydroxyanisol (BHA), tetradibutyl pentaeritryl hydroxyhidrocinnamate, benzylalconium chloride, hydrophilic substances such as penoxyethanol, benzyl alcohol, metyl paraben, propyl paraben, imidazolidinyl urea, hidantoines, polyvinyl alcohol, ethyl alcohol., octyl methoxycinnamate, sunflower-seed extract, lycopene extracted from tomato, OPC (Palmitoyl hydroxypropyl triomonium amilopectine/glycerin crospolymer, lecitin, grape-seed extract) glycospheres and derivatives thereof.

In the preferred embodiments of the cosmetic compositions of the present invention, butyl hydroxy toluene is used as an antioxidant agent in an amount ranging from 0.01% to 1.00% by weight, preferably from 0.01% to 0.20% by weight, based on the total weight of the composition.

### Fragrance

It is optional to add perfume or fragrance selected from a variety of possible substances to compositions of this kind. The amount of fragrance to be added to the cosmetic compositions of the present invention, preferably ranges from 0.01% to 30.00%, more preferably from 0.05% to 5.00% by weight, based on the total weight of the composition.

### Vehicle

Water is the base of several possibilities of cosmetic compositions prepared from the already-described base cosmetic composition, acting as a vehicle for the other components. The compositions of the present invention comprise water, preferably demineralized or distilled at an adequate percentage (q.s.p.) to reach 100% of the formula, based on the total weight of the present composition. Of course, other cosmetically acceptable vehicles may be used in the present invention. In the examples of the composition that are described later, ethyl alcohol, cyclomethicone and dicaprylyl carbonate are also used as a vehicle,.

### Other Optional Components

In order to impart to the cosmetic compositions of the present invention some desirable characteristic that is not achieved with the cited components, optional components that are compatible with their properties may be added. Some of these compounds that may be added to the composition are:
active principles (either encapsulated or not): they may be lipophilic or hydrophilic such as seaweed extract, combination of palmitoyl hydroxypropyl triomonium aminopectine, glycerin crosspolymer, lecitin and grape-seed extract, bisabolol (anti-inflammatory active), D-pantenol (conditioning active), tocopherol (vitamin E), preferably in talaspheres, combination of retinol (vitamin A) and tocopherol (vitamin E) preferably in talaspheres, ascorbic acid (vitamin C) preferably ion talaspheres, ergocalcipherol (vitamin D), biosaccharide gum 2 and biosaccharid gum 3 and other active sunscreen commonly added to compositions of products to be used topically or on the hair;
   - bacteriostatics, bactericidal or antimicrobicidal;
   - stabilizing agents such as sodium chloride;
   - dyes;
   - plant extracts: chamomile, rosemary, thyme, calendula, carrot extract, common juniper extract, gentian extract, cucumber extract;
   - skin or hair conditioning agents;
   - hair dyes;
   - optical diffusers; and
   - other cosmetically acceptable components that are compatible with the base composition.

### Examples of Composition

### Example 1 - Sport SPF 15 Protecting Hydrating Emulsion

The composition exemplified below has been prepared by fo-Ilowing these steps:
1 - add disodic EDTA (Phase B) to demineralized water (Phase A) and stir until complete solubilization is achieved;
2 - add carbomer and xanthan gum (Phase C) and stir until complete dispersion is achieved;
3 - add glycerin (Phase D) and stir until complete solubilization is achieved;
4 - heat the mixture under constant stirring up to the temperature of 85°C;
5 - separately, mix dicaprilyl carbonate, C₁₂-C₁₅ alkyl benzoate, dimethicone, trimethylsiloxysilicate, PEG-8 dimethicone, stearyl dimethicone, 2-ethylhexyloxyphenol methoxyphenyl triazine benzophenone 3, BHT (Phase E) and heat under constant stirring up to the temperature of 85°C;
6 - at the temperature of 85°C, add the Phase E to the mixture;
7 - add potassium cetylphosphate (Phase F) and triethanolamine previously solubilized in demineralized water (Phase G);
8 - stir until the emulsion is formed;
9 - cool the mixture down to the temperature of 25°C;
10 - add cyclomethicone, cyclopentasiloxane and dimethiconol (Phase H) and vitamin E acetate and lycopene extracted from tomato (Phase I) previously solubilized;
11 - add glycospheres of OPC (Palmitoyl hydroxypropyl triomonium amilopectine/glycerin crospolymer, lecitin, grape-seed extract), sunflower-seed extract, mixture of PEG-5 cocoate, PEG-8 dicocoate and iodopropinyl butyl carbamate and PEG-4 and phenoxyethanol one by one (Phase J); and
12 - stir until complete homogenization is achieved.

| | Component | Amount (% by weight) |
|---|---|---|
| A | Demineralized water | 61.8280 |
| B | Disodic EDTA | 0.10 |
| C | Carbomer | 0.35 |
| C | Xanthan gum | 0.20 |
| D | Glycerin | 5.00 |
| E | Dicaprilyl carbonate | 2.00 |
| E | C₁₂-C₁₅ alkyl benzoate | 5.00 |
| E | Dimethicone and trimethylsiloxysilicate | 2.00 |
| E | PEG-8 dimethicone | 1.00 |
| E | Dimethicone stearyl | 1.00 |
| E | 2-ethylhexyl p-methoxycinnamate | 7.00 |
| E | 4-butyl-4-methoxydibenzoylmethane | 1.00 |
| E | Benzophenone 3 | 1.50 |
| E | Bis-ethylhexyloxyphenol methoxyphenyl triazine | 1.00 |
| E | BHT | 0.05 |
| F | Potassium cetylphosphate | 2.00 |
| G | Demineralized water | 3.00 |
| G | Triethanolamine | 0.50 |
| H | Cyclomethicone | 2.00 |
| H | Cyclopentasiloxane and dimethiconol | 1.00 |
| I | Vitamin E acetate | 0.50 |
| I | Lycopene extracted from tomato | 0.002 |
| J | Glycospheres of OPC ((Palmitoyl hydroxypropyl triomonium amilopectine/glycerin crospolymer, lecitin, grape-seed extract) | 0.02 |
| J | Sunflower-seed extract | 0.25 |
| J | PEG-5 cocoate, PEG-8 dicocoate and iodopropinyl butylcarbamate and PEG-4 | 0.20 |
| J | Phenoxyethanol | 1.00 |

| | |
|---|---|
| Component | Function |
| Demineralized water | Vehicle |
| Disodic EDTA | Sequestering agent |
| Carbomer | Thickening agent |
| Xanthan gum | Thickening agent |
| Glycerin | Moistening agent |
| Dicaprilyl carbonate | Emollient |
| C₁₂-C₁₅ alkyl benzoate | Emollient |
| Dimethicone and trimethylsiloxysilicate | Emollient |
| PEG-8 dimethicone | Co-emulsifier |
| Stearyl dimethicone | Emollient |
| 2-ethylhexyl p-methoxicinnmamate | Sunscreen |
| 4-butyl-4-methoxydibenzoylmethane | Sunscreen |
| Benzophenone 3 | Sunscreen |
| Bis-ethylhexyloxyphenol methoxyphenyl triazine | Sunscreen |
| BHT | Antioxidant agent |
| Potassium cetylphosphate | Emulsifying agent |
| Demineralized water | Vehicle |
| Triethanolamine | pH adjuster |
| Cyclomethicone | Emollient |
| Cyclopentasiloxane and dimethiconol | Emollient |
| Vitamin E acetate | Antioxidant agent |
| Lycopene extracted from tomato | Antioxidant agent |
| Glycospheres of OPC (Palmitoyl hydroxypropyl triomonium amilopectine/glycerin crospolymer, lecitin, grape-seed extract) | Antioxidant agent |
| Sunflower-seed extract | Antioxidant agent |
| PEG-5 cocoate, PEG-8 dicocoate and PEG-4 iodopropinylbutylcarbamate | Preserving agent |
| Phenoxyethanol | Preserving agent |

### Tests of the cosmetic composition of the present invention

The composition used in the tests described hereinafter is that defined in example 1 - Body Hydrating Emulsion with SPF 15.

### I. TESTS FOR SAFETY

### 1 - A clinical double-blind, randomized, controlled study of the comedogenic potential

The objective of this test is to evaluate the comedogenicity of the above-cited cosmetic composition.

For this test, 10 volunteers between 24 and 45 years of age were selected (average 36 years). The volunteers with a history of chronic systemic diseases, generalized dermatological diseases, presence of nevus in the dorsal region, pregnancy or nursing were excluded. The test chosen for the cited purpose was the occlusive pacht test (known also as contact test of epicutaneous text).

The procedure of the test is as follows:
- 0.2 ml of the product was applied to the backs of the volunteers with the help of a syringe in a 8 cm² area;
- occlusion of this area was then made with non-absorbent cotton fabric and sticking plaster;
- an equivalent area was occluded, but without application of product, to serve as a control area.

This procedure was repeated 12 times in 4 weeks. After this period, follicular biopsies with the help of cyanocrylate glue, and the removed material was analyzed.

Result: the sample tested proved to be non-comedogenic.

### 2 - A clinical, mono-blind, randomized, controlled study of the phototoxicity potential and cutaneous photoallergy

The objective of this test is to evaluate whether the above-cited cosmetic composition exhibits phototoxicity and/or cutaneous photoallergy.

For this test, 25 volunteers of both sexes, between 18 and 66 years of age were selected. Volunteers with localized or generalized dermatological diseases, presence of lesions or nevus in the dorsal region, dermatoses triggered by light, use of medicament that induce photosensitivation, pregnancy and nursing were excluded.

In order to evaluate photoallergy, a filter-paper disc A with the sample of the product of 0.05 g/cm² was applied in a determined region of the back of the volunteers, this region and their eyes being protected. This procedure was repeated over 5 times. The disc was removed and the exposed region was irradiated with UVA and UVB ultraviolet lamp. The area adjacent the place of application served as a control area and was irradiated as well.

On the other hand, to evaluate phototoxicity, the procedure was equivalent, with the difference that the application took place only once and the irradiation was performed with UVA ultraviolet lamp.

Result: the cosmetic composition did not cause either phototoxicity or photoallergy, being considered approved for topical use.

### 3 - A clinical, open study of the cutaneous tolerability and of the ocular region in real conditions of use

The objective of this test is to determine the prevalence of adverse reactions in real conditions of use of the above-cited cosmetic composition.

For this purpose, 28 volunteers between 21 and 43 years of age were selected (average 36 years). Volunteers with a history of chronic systemic diseases, corneal eye diseases and dermatological conjunctive diseases either generalized or in the periocular region, participation in tests for ocular irritability on the 7 days preceding the test, occurrence of ocular irritation or ocular infection in the 48 hours preceding the test, use of contact lenses and spectacles, systemic arterial hypertension, intolerance to heat, having undergone treatment with antihistaminics, anti-inflammatories, corticosteroids or sympathomimetic medications within the period of 7 days preceding the test were excluded.

A frontal region of the face of 24 cm² area was demarcated on each of the volunteers, and the cosmetic composition was applied in an amount of 2 mg/cm² homogeneously. After application, the volunteers had a dry sauna to induce diaphoresis in two stages. Thereafter the volunteers used the cosmetic composition for 3 weeks, under supervision of a dermatologist and an ophthalmologist.

Result: in real conditions of use, no volunteer presented any cutaneous or ocular lesion related to the use of the cosmetic composition. In conditions of diaphoresis, no volunteer presented any kind of adverse ocular reaction or ocular lesion.

### 4 - A clinical, open, comparative, randomized study of the ocular irritability potential

The objective of this test is to evaluate comparatively the ocular irritability potential of the above-cited cosmetic composition, after diaphoresis and of a physiologic solution NaCl 0.9%.

For this test, 29 volunteers between 21 and 44 years of age were selected (average 32 years). Volunteers with a history of chronic systemic diseases, corneal and conjunctive ocular diseases, dermatological diseases either generalized or in the periocular region, participation in tests for ocular irritability in the 7 days preceding the test, occurrence of ocular irritation or ocular infection in the 48 hours preceding the test, use of contact lenses and spectacles, systemic arterial hypertension, intolerance to heat, having undergone treatment with antihistaminics, anti-inflammatories, cordicosteroids or sympathomimetic medications within the period of 7 days preceding the test were excluded.

A frontal region of the face of 24 cm² area was demarcated on each volunteer and the product was applied in an amount of 2 mg/cm² homogeneously. Several products were applied, randomly choosing the volunteer who would receive each of them. After application, the volunteers had a dry sauna to induce diaphoresis. Thereafter the volunteers were evaluated by an ophthalmologist.

Result: no volunteer presented ocular ardency or irritation when exposed to the conditions of intense diaphoresis. So, there is no difference between the products used in the test.

### II - TESTS FOR EFFICACY

### 1 - Evaluation of the percentage of UVA protection

The objective of this test is to determine spectrophotometrically the percentage of radiation blocking in the region of the UVA ray, according to an Australian methodology.

The cosmetic composition is applied on a tape of the Transpore type, which is kept fixed on the metallic support of the spectrophotometer. 9 different positions of the tape are read. With the data chosen, the transmittance and the blocking were calculated.

Result: the cosmetic composition exhibited a percentage of blocking higher than 90% in the 320 - 360 nm range. Therefore, it is considered to be of a broad protection spectrum.

### 2 - Evaluation of the Hydration Kinetics

The objective of this test is to analyze the kinetics of the cited cosmetic composition in order to evaluate its hydration potential.

The areas of 2.5 x 4 cm of the forearms of 10 volunteers are demarcated for application of 2 mg/cm² onto each of them. One of the areas is kept as non-treated (control area). The measurements are made with the help of an apparatus of the Corneometer CM 820 model from the company Courage and Khazaka. The measurements show that the cosmetic composition of the present invention exhibits a 22.2% higher hydration in comparison with the hydration provided by the control area.

Result: the skin hydration values remain at levels significantly higher than the control area for over 8 hours after a single application.

### 4. A clinical, open, randomized, study, parallel control over the determination of the sunscreen protection factor (SPF) before and after immersion in water

The objective of this test is to determine the sunscreen protection factor (SPF) after and before immersion into water of the above-cited cosmetic composition.

10 volunteers between 21 and 49 years of age (average of 29 years) of phototypes I, II and III were selected. Volunteers with a history of phototoxic and photoallergic reactions, use of medicaments liable to product abnormal cutaneous response, presence of sunburns, suntan, uneven skin tone, spots, nevus, seborrhoeic keratosis or excess hair on the site of the test, pregnancy or nursing were excluded.

Areas of 50 cm² were demarcated in the infrascapular region of the volunteers. Standard sunscreen and the cosmetic composition were applied in adjacent regions in amounts of 0.1 ml or 0.1g homogeneously. Thereafter, the areas were irradiated and dipped into water. This procedure was then repeated, with measurements right after application of the product of 240 minutes after immersion into water and 360 minutes of immersion into water. With the data obtained, the SPF was calculated.

Result: the product is approved as SPF 15.0.

### 5. A clinical, open, randomized study, parallel control over the determination of the sunscreen protection factor (SPF) before and after diaphoresis

The objective of this test is to determine the sunscreen protection factor before and after diaphoresis of the above-cited cosmetic composition.

10 volunteers between 27 and 42 years of age (average 33 years) of I, II, and III phototypes were selected. Volunteers with a history of phototoxic and photoallergic reactions, use of medicaments liable to product abnormal cutaneous response, presence of sunburns, suntan, uneven skin tone, spots, nevus, seborrhoeic keratosis or excess hair on the site of the test, pregnancy or nursing were excluded.

Three areas of 50 cm² were demarcated in the infrascapular region of the volunteers. A standard sunscreen and the cosmetic composition were applied in adjacent regions in the amount of 0.1 ml or 0.1g homogenously. Thereafter, the areas were irradiated and exposed to heat (diaphoresis). This procedure was then repeated, with the measurements immediately after application of the product and after 120 minutes of exposure to heat. With the results obtained, the SPF was calculated.

Result: the product is approved as SPF 15.0.

### III - SENSORIAL EVALUATION

These studies aim at evaluating the acceptance by the consumer of the cosmetic composition of the present invention. 78 male and female volunteers, between 16 and 50 years of age, phototypes I, II, III and IV, being users of photoprotectors were selected. Volunteers who had dermatological diseases or were pregnant or nursing were excluded.

The questionnaire for evaluation of performance and acceptance of the product was answered during the period of test. These evaluations were carried out in parallel with a cosmetic composition from the market, also with the appeal of resistance to water for 6 hours and resistance to sweat without definite time.

The results of the evaluations show that, during application, the consumers considered the texture of the product adequate, that is to say, non-sticky or non-oily, considered the product to be easy to spread and with ideal time of absorption/drying, and indicated that it, after application, did not leave the skin too oily.

These conclusions are reached from the following results:
- 85% of the volunteers indicated that the product exhibits an adequate texture during application, value of 16.5% better than the market composition;
- in hedonic evaluation, the acceptance index for the attribute stickiness was of 83%, value 48% better than the market composition;
- in hedonic evaluation, the acceptance index for the attribute oiliness was of 81 %, value 68% better than the market composition;
- 90% of the volunteers considered the product easy or very easy to spread, value 45% better than the market composition;
- in hedonic evaluation, the acceptance index for the attribute drying time was of 87%, value 30% better than the market composition;
- 92% of the volunteers indicated that the product does not leave the skin too oily after application, value 33% better than the market composition.

### IV - TESTS FOR STABILITY

Semi-pilot scale and industrial bench batches were analyzed for determination/evaluation of their physical-chemical parameters, as well as organoleptic parameters:
- viscosity: the samples subjected to conditions of 37°C, 5°C, 25°C, sunlight and 45°C for 1 month kept the viscosity within the specified range;
- pH: the samples subjected to the conditions of 37°C, 5°C, 25°C, sunlight and 45°C for 1 month kept the viscosity within the specified range;
- appearance: 37°C, 5°C, 25°C, sunlight and 45°C for 1 month did not present significant variations;
- color: 37°C, 5°C, 25°C, sunlight and 45°C for 1 month did not present significant variations;
- odor: 37°C, 5°C, 25°C, sunlight and 45°C for 1 month did not present significant variations.

### Example 2 - Definition and Volume Cream: Crisp Hair

This composition was prepared in a similar way as the composition of example 1 above. The difference lies in the constitution of Phase E, which in the case of example 2 comprises these compounds: mixture of dicaprilyl carbonate, C₁₂-C₁₅ alkyl benzoate, dimethicone, trimethylsiloxysilicate, PEG-8 dimethicone, stearyl dimethicone and BHT.

| Phase | Component | Amount (% by weight) |
|---|---|---|
| A | Demineralized water | 56.83 |
| B | Disodic EDTA | 0.10 |
| C | Carbomer | 0.35 |
| C | Xanthan gum | 0.20 |
| D | Glycerin | 5.00 |
| E | Dicaprylyl carbonate | 2.00 |
| E | E C12-C15 dimethicone benzoate | 5.00 |
| E | Trimethylsiloxysilicate | 2.00 |
| E | PEG-8 dimethicone | 1.00 |
| E | Stearyl dimethicone | 1.00 |
| E | BHT | 0.05 |
| F | Potassium cetylphosphate | 2.00 |
| G | Demineralized water | 3.00 |
| G | Triethanolamine | 0.50 |
| H | Cyclomethicone | 2.00 |
| H | Cyclopentasiloxane and dimethiconol | 1.00 |
| I | Vitamin E acetate | 0.50 |
| I | Lycopene extracted from tomato | 0.002 |
| J | Glycospheres of OPC (Palmitoyl hydroxypropyl trimonium amilopectine/glycerin crosspolymer, lecitin, grape-seed extract) | 0.02 |
| J | Sunflower-seed extract | 0.25 |
| J | PEG-5 cocoate, PEG-8 dicocoate and iodopropinyl butylcarbamate and PET-4 | 0.20 |
| J | Phenoxyethanol | 1.00 |

| Component | Function |
|---|---|
| Demineralized water | Vehicle |
| Disodic EDTA | Sequestering agent |
| Carbomer | Thickening agent |
| Xanthan gum | Thickening agent |
| Glycerin | Moistening agent |
| Dicaprilyl carbonate | Emollient |
| C12-C15 alkyl benzoate | Emollient |
| Dimethicone and trimethylsiloxysilicate | Emollient |
| PEG-8 dimethicone | Co-emulsifier |
| Stearyl dimethicone | Emollient |
| BHT | Antioxidant agent |
| Potassium cetylphosphate | Emulsifying agent |
| Demineralized water | Vehicle |
| Triethanolamine | pH adjuster |
| Cyclomethicone | Emollient |
| Cyclopentasiloxane and dimethiconol | Emollient |
| Vitamin E acetate | Antioxidant agent |
| Lycopene extracted from tomato | Antioxidant agent |
| Glycospheres of (Palmitoyl hydroxypropyl trimonium amilopectine/glycerin crosspolymer, lecitin, grape-seed extract) | Antioxidant agent |
| Sunflower-seed extract | Antioxidant agent |
| PEG-5 cocoate, PET-8 dicocoate and iodopropinyl butylcarbamate PEG-4 | Preserving agent |
| Phenoxyethanol | Preserving agent |

### Test of the Cosmetic Composition of Example 2

The composition used in the tests described below is that defined in example 2 - Definition and Volume Cream: Crisp Hair

### I -TEST FOR EFFICACY

### 1 - Evaluation of Curl Retention

The objective of this test is to evaluate the curl retention potential of the compositions in a long-term study and in conditions of high relative humidity.

The measurement of the capacity of retaining curls is based on the analysis of the image (photographs) of previously treated and modeled locks of hair that remain under high relative humidity. The photos are registered every hour (totalizing 8 hours) and then the lengths of the locks are measured. The product capable of maintaining the shortest length of the hair, with respect to both the initial lock size (expression A) and the initial curl size (expression B) over time will have the greatest power of retaining curls, that is to say, it will have the least loss of hair shaping (in percentage).

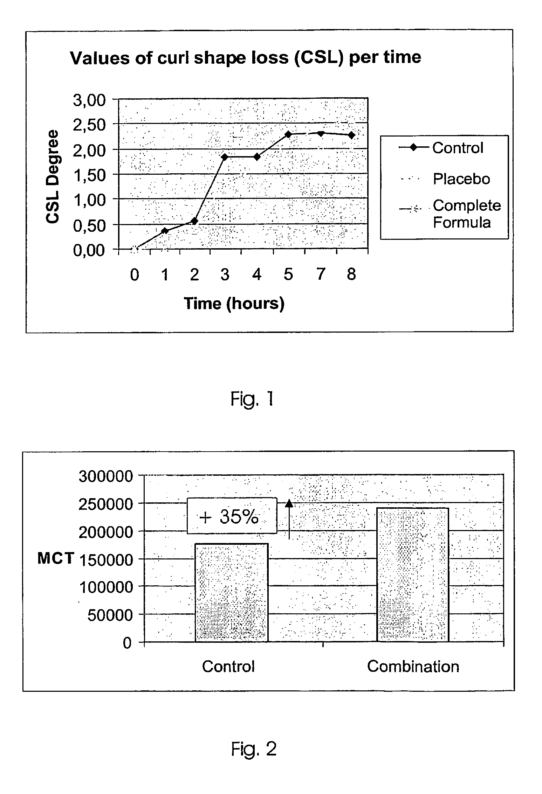

For this test, control locks (without application of product), locks with application of placebo (components of the filmogenic and emulsifying systems: cyclopentasiloxane, dimethiconol, dimethicone, trimethylsiloxysilicate and stearyl dimethicone and PEG-8 dimethicone) and locks with application of the complete formula, described in Example 2 have been used. Note: Figure 1: A graph illustrating the values of loss of curl shaping (LCS) over time.

Result: As can be concluded from Figure 1, the best product for retaining curls is the cosmetic composition of the present invention, described in Example 2, which provides retention of curls significantly greater than the retention achieved in the control or that provided by placebo, for over 8 hours after a single application in conditions of high relative humidity (UR_{min.} = 85%).

### Example 3 - Hair Protection Fluid

The cosmetic composition exemplified below has been prepared according to the following steps:
1 - heating dimethicone, trimethylsiloxysilicate, PEG-8 dimethicone, stearyl dimethicone (Phase A) up to the temperature of 75°C;
2 - cooling the mixture down to the temperature of 50°C;
3 - adding cyclopentasiloxane and dimethiconol (Phase B) and stirring until complete solubilization is reached.

| Phase | Component | Amount (% by weight) |
|---|---|---|
| A | Dimethicone and trimethylsiloxysilicate | 2.00 |
| A | PEG-8 dimethicone | 1.00 |
| A | Stearyl dimethicone | 1.00 |
| B | Cyclopentasiloxane and dimethiconol | 1.00 |

| Phase | Function |
|---|---|
| Dimethicone and trimethylsiloxysilicate | Emollient |
| PEG-8 dimethicone | Co-emulsifier |
| Stearyl dimethicone | Emollient |
| Cyclopentasiloxane and dimethiconol | Emollient |

### Test of the Cosmetic Composition of the Present Invention

The composition used in the tests described below is that defined in Example 3 - Hair Protection Fluid.

### I - TEST FOR EFFICACY

### 1 - Measurement of the Mechanical Properties of Hair

The objective of this test is to obtain values of parameters relating to the mechanical properties of individual hairs by applying a known formula for obtaining the applied tension curve versus deformation/lengthening undergone by the hair. Control individual hairs (without treatment) and hairs treated with the products of combination of silicones (composition of Example 3), Sport formula (Example 1 without the sunscreen agents) and placebo of this same formulation have been used.

The individual hairs were subjected to the adequate treatments.

The equipment Instron was used for obtaining the measurements of tension versus deformation.

The hairs were conditioned in a room with controlled humidity between 55 and 65% of relative humidity.

30 individual hairs were treated with the cosmetic composition of the present invention, constituted by the base composition at defined concentrations, and 30 control hairs.

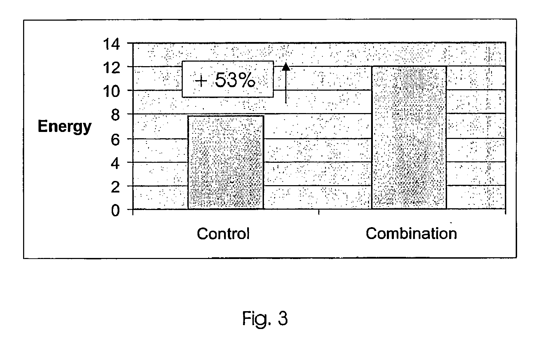

The final results are expressed in energy at the breaking point (J) and in the tension on the maximum load (MPa), as can be seen in the graphs of Figures 2 and 3. With the values of the graph, the decrease or increase in energy and tension for breaking an individual hair was calculated (in percentage).

Locks of hair were separated for each treatment to be evaluated. In the application, a proportion of 1 g of product for 6 g of lock was used. There was no rinsing.

Each individual hair, having its diameter measured with the help of a micrometer, was kept suspended by two pneumatic claws separated by a distance of 5 cm. The constant velocity applied in the upper claw for stretching the hair was of 10 mm/min. After rupture, the equipment was programmed for causing the upper claw to return to the initial position.

The software registered various parameters for each individual hair, such as young module, rupture energy, tension in function of the deformation undergone by the individual hair. The values measured are shown in the graphs of figures 2 and 3.

Notes: Figure 2 shows a graph that evaluates the Increase in maximum load tension (MLT) on individual hairs treated in comparison with untreated ones (control hairs);

Figure 3 shows a graph that evaluates the energy at the rupture point on treated hairs in comparison with untreated hairs (control hairs).

### Results:

The treatment with the Hair Protection Fluid (composition of Example 3) has provided an increase in the energy necessary for rupture of the individual hair (35%), which results in a strengthening of the individual hair (greater resistance).

Preferred embodiments having been described, one should understand that the scope of the present invention embraces other possible variations, being limited only by the contents of the accompanying claims, which include the possible equivalents.

### Other Examples of Composition

### Example 4 - Sport SPF 30 Hydrating Protecting Emulsion

| Component | Amount (% by weight) |
|---|---|
| Demineralized water | 40.3280 |
| Disodic EDTA | 0.10 |
| Glycerin | 5.00 |
| Seaweed extract | 2.00 |
| Crosspolymers of acrylates/C₁₀₋₃₀ alkyl acrylate | 0.30 |
| Xanthan gum | 0.05 |
| Stearyl dimethicone | 1.20 |
| PEG-8 dimethicone | 1.20 |
| 2-ethylhexyl p-methoxycinnamate | 9.00 |
| 4-butyl-4-methoxydibenzoylmethane | 3.00 |
| Benzophenone 3 | 4.00 |
| Dicaprilyl carbonate | 6.00 |
| Octyl triazone | 3.00 |
| Dimethicone and trimethylsiloxysilicate | 2.70 |
| Cyclopentasiloxane and dimethiconol | 1.50 |
| Cyclomethicone | 3.00 |
| BHT | 0.05 |
| Dicaprylic ether | 3.00 |
| Aluminum octenylsuccinate starch | 6.00 |
| Potassium cetylphosphate | 2.00 |
| Nylon 12 | 1.00 |
| Demineralized water | 3.00 |
| Triethanolamine | 0.60 |
| Vitamin E acetate | 0.50 |
| Lycopene extracted from tomato | 0.002 |
| Glycospheres of OPC (Palmitoyl hydroxypropyl trimonium amilopectine/glycerin crosspolymer, lecitin, grape-seed extract) | 0.02 |
| Sunflower-seed extract | 0.25 |
| PEG-5 cocoate, PEG-8 dicocoate and iodopropinyl butylcarbamate PEG-4 | 0.20 |
| Phenoxyethanol | 1.00 |

| Component | Function |
|---|---|
| Demineralized water | Vehicle |
| Disodic EDTA | Sequestering agent |
| Glycerin | Moistening agent |
| Seaweed extract | Active |
| Crosspolymers of acrylates/C10-30 alkyl acrylate | Thickening agent |
| Xanthan gum | Thickening agent |
| Stearyl dimethicone | Emollient |
| PEG-8 dimethicone | Co-emulsifier |
| 2-ethyhexyl p-methoxycinnamate | Sunscreen |
| 4-buty-4-methoxydibenzoylmethane | Sunscreen |
| Benzophenone 3 | Sunscreen |
| Dicaprilyl carbonate | Emollient |
| Octyl triazone | Sunscreen |
| Dimethicone and trimethylsiloxysilicate | Emollient |
| Cyclopentasiloxane and dimethiconol | Emollient |
| Cyclomethicone | Emollient |
| BHT | Antioxidant agent |
| Aluminum octenylsuccinate starch | Oiliness adsorbing agent |
| Potassium cetylphosphate | Emulsifier |
| Nylon 12 | Oiliness adsorbing agent |
| Demineralized water | Vehicle |
| Triethanolamine | pH adjuster |
| Vitamin E acetate | Antioxidant agent |
| Lycopene extracted from tomato | Antioxidant agent |
| Glycospheres of OPC (Palmitoyl hydroxypropyl trimonium amilopectine/glycerin crosspolymer, lecitin, grape-seed extract) | Antioxidant agent |
| Sunflower-seed extract | Antioxidant agent |
| PEG-5 cocoate, PEG-8 dicocoate and iodopropinyl butylcarbamate PEG-4 | Preserving agent |
| Phenoxyethanol | Preserving agent |

### Example 5 - SPF 30 Protecting Gel

| Component | Amount (% by weight) |
|---|---|
| Neutral 96° ethyl alcohol | Qsp 100 |
| Hydroxypropylcellulose | 1.10 |
| 2-ethylhexyl p-methoxycinnamate | 9.00 |
| Benzophenone 3 | 4.00 |
| 4-butyl-4-methoxydibenzoylmethane | 2.00 |
| Octyl triazone | 3.00 |
| BHA | 0.05 |
| Cetyl lactate | 1.00 |
| Stearyl dimethicone | 1.00 |
| PEG-8 dimethicone | 0.80 |
| Dimethicone and trimethylsiloxysilicate | 1.70 |
| Cyclopentasiloxane and dimethiconol | 0.75 |
| Vitamin E acetate | 0.50 |
| Lycopene extracted from tomato | 0.002 |
| Glycospheres of OPC (Palmitoyl hydroxypropyl trimonium amilopectine/glycerin crosspolymer, lecitin, grape-seed extract) | 0.02 |
| Sunflower-seed extract | 0.25 |
| PEG-5 cocoate, PEG-8 dicocoate and iodopropinyl PEG-4 butylcarbamate | 0.20 |
| Phenoxyethanol | 1.00 |

| Component | Function |
|---|---|
| Neutral 96° ethyl alcohol | Vehicle |
| Hydroxypropylcellulose | Thickening agent |
| 2-ethylhexyl p-methoxycinnamate | Sunscreen |
| Benzophenone 3 | Sunscreen |
| 4-butyl-4-methoxydibenzoylmethane | Sunscreen |
| Octyl triazone | Sunscreen |
| BHA | Antioxidant agent |
| Cetyl lactate | Emollient |
| Stearyl dimethicone | Emollient |
| PEG-8 dimethicone | Co-emulsifier |
| Dimethicone and trimethylsiloxysilicate | Emollient |
| Cyclopentasiloxane and dimethiconol | Emollient |
| Vitamin E acetate | Antioxidant agent |
| Lycopene extracted from tomato | Antioxidant agent |
| Glycospheres of OPC (Palmitoyl hydroxypropyl trimonium amilopectine/glycerin crosspolymer, lecitin, grape-seed extract) | Antioxidant agent |
| Sunflower-seed extract | Antioxidant agent |
| PEG-5 cocoate, PEG8-dicocoate and iodopropinyl PEG-4 butylcarbamate | Preserving agent |
| Phenoxyethanol | Preserving agent |

### Example 6 - SPF 30 Lip Protector

| Component | Amount (% by weight) |
|---|---|
| Dicaprilyl carbonate | 39.74 |
| Microcrystalline wax | 16.80 |
| Carnauba wax | 5.76 |
| Shea butter | 3.50 |
| Vitamin E acetate | 0.50 |
| Bee wax | 3.40 |
| Ozokerite wax | 3.75 |
| Glycerin behenate | 1.00 |
| Stearyl dimethicone | 0.50 |
| PEG-8 dimethicone | 0.70 |
| Dimethicone and trimethylsiloxysilicate | 2.30 |
| Cyclopentasiloxane and dimethiconol | 1.20 |
| 2-ethylhexyl p-methoxicinnamate | 7.50 |
| 4-butyl-4-methoxydibenzoylmethane | 4.00 |
| Benzophenone 3 | 6.00 |
| Octyl triazone | 3.00 |
| Methyl parabene | 0.20 |
| Propyl parabene | 0.10 |
| BHT | 0.05 |

| Component | Function |
|---|---|
| Dicaprilyl carbonate | Emollient |
| Microcrystalline wax | Emollient |
| Carnauba wax | Emollient |
| Shea butter | Emollient |
| Vitamin E acetate | Antioxidant agent |
| Bee wax | Emollient |
| Ozokerite wax | Emollient |
| Glycerin behenate | Emollient |
| Stearyl dimethicone | Emollient |
| PET-8 dimethicone | Co-emulsifier |
| Dimethicone and trimethylsiiloxysilicate | Emollient |
| Cyclopentasiloxane and dimethiconol | Emollient |
| 2-ethylhexyl p-methoxycinnamate | Sunscreen |
| 4-butyl-methoxydibenzoylmethane | Sunscreen |
| Benzophenone 3 | Sunscreen |
| Octyl triazone | Sunscreen |
| Methyl parabene | Preserving agent |
| Propyl parabene | Preserving agent |
| BHT | Antioxidant agent |

### Example 7 - SPF 30 Baby Protecting Emulsion

| Component | Amount (% by weight) |
|---|---|
| Cyclomethicone | 24.80 |
| Titanium dioxide | 16.00 |
| Cyclomethicone and dimethicone copolyol | 10.00 |
| Cetyl dimethicone copolyol | 5.00 |
| Triglyceryl-4 isostearate | 2.00 |
| Demineralized water | 15.00 |
| Xanthan gum | 0.10 |
| Disodic EDTA | 0.05 |
| Sodium chloride | 1.50 |
| Demineralized water | 15.5410 |
| Butyleneglycol | 3.00 |
| Polysorbate 20 | 0.10 |
| Triethanolamine | 0,.009 |
| Phenoxyethanol | 1.00 |
| Vitamin E acetate | 0.50 |
| PEG-5 cocoate and PEG-8 dicocoate and iodopropinyl butylcarbamate and PEG-4 | 0.20 |
| Stearate dimethicone | 1.00 |
| PEG-8 dimethicone | 0.80 |
| Dimethicone and trimethylsiloxysilicate | 2.20 |
| Cyclopentasiloxane and dimethiconol | 1.20 |

| Component | Function |
|---|---|
| Cyclomethicone | Emollient |
| Titanium dioxide | Sunscreen |
| Cyclomethicone and dimethicone copolyol | Emulsifier |
| Cetyl dimethicone copolyol | Co-emulsifier |
| Triglyceryl-4 isostearate | Emollient |
| Demineralized water | Vehicle |
| Xanthan gum | Thickening agent |
| Disodic EDTA | Sequestering agent |
| Sodium chloride | Stabilizing agent |
| Demineralized water | Vehicle |
| Butyleneglycol | Moistening agent |
| Polysorbate 20 | Co-emoulsifier |
| Triethanolamine | pH adjuster |
| Phenoxyethanol | Preserving agent |
| Vitamin E acetate | Antioxidant agent |
| PEG-5 cocoate and PEG-8 dicocoate and iodopropinyl butylcarbamate and PEG-4 | Preserving agent |
| Stearyl dimethicone | Emollient |
| PEG-8 dimethicone | Co-emulsifier |
| Dimethicone and trimethylsiloxysilicate | Emollient |
| Cyclopentasiloxane and dimethiconol | Emollient |

### Example 8 - Hair Protecting Sunscreen

| Component | Amount (% by weight) |
|---|---|
| Demineralized water | 61.8280 |
| Disodic EDTA | 0.10 |
| Carbomer | 0.35 |
| Xanthan gum | 0.20 |
| Glycerin | 5.00 |
| Dicaprilyl carbonate | 2.00 |
| C₁₂₋₁₅ alkyl benzoate | 5.00 |
| Dimethicone and trimethylsiloxysilicate | 2.00 |
| PEG-8 dimethicone | 1.00 |
| Stearyl dimethicone | 1.00 |
| BHT | 0.05 |
| Cinnamidopropyltrimonium chloride | 1.0 |
| Dimethylpabamidopropyl laurdimonium tosylate | 0.5 |
| Polyquaternium 37 and propyleneglycol | 4.00 |
| Demineralized water | 3.00 |
| Triethanolamine | 3.00 |
| Cyclomethicone | 2.00 |
| Cyclopentasiloxane and dimethiconol | 1.00 |
| Vitamin E acetate | 0.50 |
| Lycopene extracted from tomato | 0.002 |
| Glycospheres of OPC (Palmitoyl hydroxypropyl trimonium amilopectine/glycerin crosspolymer, lecitin, grape-seed extract) | 0.02 |
| Sunflower-seed extract | 0.25 |
| PEG-5 cocoate and PEG-8 dicocoate and iodopropinyl butylcarbamate and PEG-4 | 0.20 |
| Phenoxyethanol | 1.00 |

| Component | Function |
|---|---|
| Demineralized water | Vehicle |
| Disodic EDTA | Sequestering agent |
| Carbomer | Thickening agent |
| Xanthan gum | Thickening agent |
| Glycerin | Moistening agent |
| Dicaprilyl carbonate | Emollient |
| C₁₂₋₁₅ alkyl benzoate | Emollient |
| Dimethicone and trimethylsiloxysilicate | Emollient |
| PEG-8 dimethicone | Co-emulsifier |
| Stearyl dimethicone | Emollient |
| BHT | Antioxidant agent |
| Cinnamidopropyltrimonium chloride | Sunscreen |
| Dimethylpabamidopropyl laurdimonium tosylate | Sunscreen |
| Polyquaternium 37 and propyleneglycol | Emulsifier |
| Demineralized water | Vehicle |
| Triethanolamine | pH adjuster |
| Cyclomethicone | Emollient |
| Cyclopentasiloxane and dimethiconol | Emollient |
| Vitamin E acetate | Antioxidant agent |
| Lycopene extracted from tomato | Antioxidant agent |
| Glycospheres of OPC (Palmitoyl hydroxypropyl trimonium amilopectine/glycerin crosspolymer, lecitin, grape-seed extract) | Antioxidant agent |
| Sunflower-seed extract | Antioxidant agent |
| PEG-5 cocoate and PEG-8 dicocoate and iodopropinyl butylcarbamate and PEG-4 | Preserving agent |
| Phenoxyethanol | Preserving agent |

## Claims

1. A cosmetic composition **characterized by** comprising a filmogenic system containing at least cyclopentasiloxane, dimethiconol, dimethicone, trimethylsiloxysilicate and stearyl dimethicone and an emulsifying system containing at least PEG-8 dimethicone.

2. A cosmetic composition according to claim 1, **characterized in that** the amount of the filmogenic system ranges from 0.3% to 92,0% by weight.

3. A cosmetic composition according to claim 3, **characterized in that** the amount of the filmogenic system ranges from 0.5% to 50.0% by weight.

4. A cosmetic composition according to claim 1, **characterized in that** the amount of the emulsifying system ranges from 0.1% to 28.0% by weight.

5. A cosmetic composition according to claim 4, **characterized in that** the amount of the emulsifying system ranges from 0.3% to 12.0% by weight.

6. A cosmetic composition according to claim 1, **characterized in that** the filmogenic system further comprises an optional filmogenic agent selected from the group consisting of cyclomethicone, alkyldimethicone, dimethicone copolyol, phenyl trimethicone, caprilyl trimethicone, aminofunctional silicones, phenyl modified silicones, phenyl trimethicone, alkyl modified silicones, dimethyl and diethyl polysiloxane, mixed C₁-C₃₀ alkyl polysiloxane, dimethyl siloxane, o-polymethylsiloxane, α-methyl-ω-methoxypolymethylsiloxane, polyoxydimethylsilylene, polydimethyl silicone oil, silicone elastomers such as cyclomethicone and dimethicone crosspolymer, vinyl dimethicone and dimethicone crosspolymer, dimethicone and dimethicone crosspolymer and cyclopentasiloxane and dimethicone crosspolymer, polymers such as polyvinyl pyrrolidone and polyvinyl alcohols and combinations thereof.

7. A cosmetic composition according to claim 1, **characterized in that** the emulsifying system comprises an additional emulsifying system selected from the group consisting of: soybean lecitin, nonyl phenol ethoxylate, sodium or potassium alkyl phosphates, diethanolamine cetyl phosphate, dimethicone copolymers such as modified polyoxyalkylene dimethylpolysiloxanes, methyl glucose, propylglyceryl glucose, ethoxylated glycols, non-ethoxylated glycols, ethoxylated fatty acids, non-ethoxylated fatty acids, ethoxylated sucrose, non-ethoxylated sucrose, ethoxylated pentaeritritol, non-ethoxylated pentaeritritol, ethoxylated alkyl glycosides, non-ethoxylated alkyl glycosides, ethoxylated alkyl polyglycosides, propyleneglycol, esters such as glyceryl stearate, ethoxylated sorbitan esters, non-ethoxylated sorbitan esters, such as sorbitan stearate , ethoxylated sorbitan stearate, polysorbate 80, polysorbate 65, polysorbate 60, polysorbate 40, polysorbate 20, polyquaternium 37, glycol stearate, PEG-2 stearate, PEG-10 stearate, PEG-30 stearate, PEG-40 stearate, PEG-100 stearate, PEG-150 stearate, PEG-1000 stearate, sorbitan palmitate, propylene glycol stearate, propylene glycol PEG-25 stearate, sorbitan oleate, methyl glucose sesquistearate, fatty alcohols such as cetyl alcohol, stearyl alcohol, cetearyl alcohol, propoxylated and/or ethoxylated fatty alcohols, synthetic polymers formed by carboxyvinylic polymers and copolymers, acrylates, methacrylates, alkyl acrylates, acrylates / C₁₀-C₃₀ alkyl crosspolymer, acrylamides, taurates, ethylene oxide and propylene oxide copolymers, and combinations thereof and derivatives thereof.

8. A cosmetic composition according to claim 1, **characterized by** comprising a sunscreen system in an amount ranging from 0.1% to 50.0% by weight.

9. A cosmetic composition according to claim 8, **characterized by** comprising a sunscreen system in an amount ranging from 0.1% to 30.0% by weight.

10. A cosmetic composition according to claim 9, **characterized in that** the sunscreen system is composed of a combination between: benzylidene camphor, isophthalylidene camphor, terephthalyllidene camphor, cinnamic acid, salicylic acid, benzoic acid, p-aminobenzoic acid, substituted hydrozibenzophenones, substituted dibenzoylmethane, benzotriazol such as 2-arylbenzotriazol, 2-arylbenzimidazol, 2-arylbenzofuranes, 2-arylbenzoxazol, 2-arylindol, mono-phenylcyanoacrylates, diphenylcyanoacrylates, triazine derivatives such as hydroxyphenyltriazine compounds and benzotriazol derivatives, amides containing a vinyl group, cinnamic acid derivatives, sulfonated benzimidazoles, diphenylmalonitriles, oxalylamides, camphor derivatives, salicylic acid derivatives such as 2-ethylhexyl salicylates, homosalates and isopropyl salicylates, diphenylacrylates, benzophenone derivatives such as benzophenone-2, benzophenone-3 and benzophenone-4, PABA such as 2-ethylhexyl 4-dimethylaminobenzoate, N,N,N-trimethyl-4-(2-oxobom-3-ylideneomethyl) anilinomethyl sulfate, 3,3'-(1,4-phenylenedimethylene) bis (7,7-dimethyl-2-oxo-bicyclo-(2.2.1) 1-hepthylmethanosulfonic acid; 1-(4-terbutylphenyl)-3-(4-methoxyphenyl) propane-1, 3-dione; α-(2 oxobom-3-ylidene) toluene-4-sulfonic acid and its salts of potassium, sodium and triethanolamine; 2-ethylexyl 2-cyano-3, 3'-diphenylacrylate; 2-ethoxyethyl 4-methoxycinnamate; 2,2'-dihydroxy-4-methoxybenzophenone; methyl anthanilate; triethanolamine salicylate; 2,2',4,4' tetrahydroxybenzophenone; 2-phenylbenzimidazol-5 sulfonic acid and its salts of potassium, sodium and triethanolamine; 2-ethylhexyl 4-methoxycinnamate; 2-hydroxy-4-methoxybenzophenone (oxybenzone); 2-hydroxy-4-methoxybenzophenone sulfonic acid and its salt of sodium (sulisobenzone and sodic sulisobenzone); 4 aminobenzoic PABA acid; homomethyl salicylate; N-{(2 and 4) [(2 oxobom-3-ylidene) methyl] benzyl} acrylamide polymer; titanium dioxide (with or without lipophilic coating); ethyl N-ethoxy-4-aminobenzoate; 2-ethylhexyl 4-dimethyl aminobenzoate; 2-ethylhexyl salicylate; isopentyl 4-methoxycinnamate;3-(4'-methylbenzylidene-d-1-camphor; 3-benzylidene camphor; 2,4,6- trianiline-(p-carbo-2-ethyl-hexyl-l'-oxy)-1,2,5-triazine octyl; zinc oxide (with or without lipophilic coating); 2-(2H-benzotriazol-2-yl)-4-methyl-6-{2-methyl-3-(1,3,3,3-tetramethyl-1-((trimethylsilyl) oxy) disiloxanyl) propyl} phenol; benzoic acid; 4,4'-[[6-[[4-[[(1,1-dimethyl-ethyl] amino] carbonyl] phenyl] amino]-1,3k,5-triazine-2,4-diyl] diimino] bis, bis (2 ethylhexyl); 2,2'-methylene-bis-6-(2H-benzotriazol-2-yl)-4-(tetramethyl butyl)-1,1,3,3-phenol; methylene bis-benzotriazolyl tetraethyl butyl phenol; monosodic salt of 2,2'-bis-(1,4-phenylene)-IH-benzimidazol-4, 6 disulfonic acid; (1, 3, 5)-triazine-2, 4-bis {[4-(2-ethyl-hexyloxy)-2-hydroxy]-6-(4-methoxyphenyl); bis-ethylhexyloxyphenol methoxyphenyl triazine; methylene bis-benzotriazolyl tetramethylbutyl-phenol; butyl methoxydibenzoylmethane, 2-ethylhexyl p-methoxycinnamate, methylene bis-benzotriazolyl tetramethylbutylphenol, 4-butyl-methoxydibenzolmethane, bis-ethylhexyloxyphenol methoxyphenyl triazine, octyl triazone, titanium dioxide and derivatives thereof and combinations thereof.

11. A cosmetic composition according to claim 10, **characterized in that** the sunscreen system is composed of benzophenone-3, butyl methoxydibenzoylmethane, 2-ethylhexyl p-methoxycinnamate and bis-ethylhexyloxyphenol methoxyphenyl triazine.

12. A cosmetic composition according to claim 10, **characterized in that** the sunscreen system is composed of cinnamidopropyltrimonium chloride and dimethylpabamidopropyl laurdimonium tosylate.

13. A cosmetic product **characterized in** comprising the cosmetic composition as defined in any one of the claims 1 to 12.

14. A cosmetic product according to claim 13, **characterized by** being a body sunscreen for skin of adults and children.

15. A cosmetic product according to claim 13, **characterized by** being a body or face hydrating product.

16. A cosmetic product according to claim 13, **characterized by** being a self-tanning product.

17. A cosmetic product according to claim 13, **characterized by** being an insect repellent product.

18. A cosmetic product according to claim 13, **characterized by** being a pharmaceutical preparation.

19. A cosmetic product according to claim 13, **characterized by** being a sunscreen for hair.

20. A cosmetic product according to claim 13, **characterized by** being a non-rinsable hair conditioner.

21. A cosmetic product according to claim 13, **characterized by** being a rinsable hair preparation.

22. An use of the cosmetic composition as defined in claims 1 to 12, **characterized by** being for producing a cosmetic product for care or protection of the skin or hair.

23. An use according to claim 22, **characterized by** the fact that the cosmetic product is a sunscreen for skin of adults and children.

24. An use according to claim 22, **characterized by** the fact that the cosmetic product is a body or face hydrating product.

25. An use according to claim 22, **characterized by** the fact that the cosmetic product is a self-tanning product.

26. An use according to claim 22, **characterized by** the fact that the cosmetic product is an insect repellent product.

27. An use according to claim 22, **characterized by** the fact that the cosmetic product is a pharmaceutical preparation.

28. An use according to claim 22, **characterized by** the fact that the cosmetic product is a sunscreen for hair.

29. An use according to claim 22, **characterized by** the fact that the cosmetic product is a non-rinsable hair conditioner.

30. An use according to claim 22, **characterized by** the fact that the cosmetic product is a rinsable hair preparation.

## Patentansprüche

1. Kosmetische Zusammensetzung, **dadurch gekennzeichnet, dass** sie enthält:
ein filmbildendes System, mindestens enthaltend Cyclopentasiloxan, Dimethiconol, Dimethicon, Trimethylsiloxysilicat und Stearyldimethicon; und ein emulgierendes System, mindestens enthaltend PEG-8-dimethicon.

2. Kosmetische Zusammensetzung nach Anspruch 1, **dadurch gekennzeichnet, dass** die Menge des filmbildenden Systems 0,3 bis 92.0 Gew.-% beträgt.

3. Kosmetische Zusammensetzung nach Anspruch 2, **dadurch gekennzeichnet, dass** die Menge des filmbildenden Systems 0,5 bis 50,0 Gew.-% beträgt.

4. Kosmetische Zusammensetzung nach Anspruch 1, **dadurch gekennzeichnet, dass** die Menge des emulgierenden Systems 0,1 bis 28,0 Gew.-% beträgt.

5. Kosmetische Zusammensetzung nach Anspruch 4, **dadurch gekennzeichnet, dass** die Menge des emulgierenden Systems 0,3 bis 12,0 Gew.-% beträgt.

6. Kosmetische Zusammensetzung nach Anspruch 1, **dadurch gekennzeichnet, dass** das filmbildende System weiterhin ein fakultatives filmbildendes Mittel, ausgewählt aus der Gruppe, bestehend aus Cyclomethicon, Alkyldimethicon, Dimethicon-Copolyol, Phenyltrimethicon, Caprilyltrimethicon, aminofunktionellen Siliconen, phenylmodifizierten Siliconen, Phenyltrimethicon, alkylmodifizierten Siliconen, Dimethyl- und Diethylpolysiloxan, gemischtem C₁ - C₃₀- Alkylpolysiloxan, Dimethylsiloxan, o-Poly- methylsiloxan, α-Methyl- ω-methoxypolymethylsiloxan, Polyoxydimethylsilylen, Polydimethylsiliconöl, Siliconelastomeren, wie Cylomethicon- und Dimethicon-Crosspolymer, Vinyldimethicon- und Dimethycon-Crosspolymer, Dimethicon-und Dimethicon-Crosspolymer und Cyclopentasiloxan- und Dimethicon- Crosspolymer, Polymeren, wie Polyvinylpyrrolidon und Polyvinylalkoholen und deren Kombinationen enthält.

7. Kosmetische Zusammensetzung nach Anspruch 1, **dadurch gekennzeichnet, dass** das emulgierende System ein zusätzliches emulgierendes System, ausgewählt aus der Gruppe, bestehend aus Sojabohnen-Lecithin, Nonylphenolethoxylat, Natrium- oder Kaliumalkylphosphaten, Diethanolamincetylphosphat, Dimethicon-Copolymeren, wie modifizierten Polyoxyalkylen-Dimethylpolysiloxanen, Methylglucose, Propylglycerylglucose, ethoxylierten Glykolen, nicht-ethoxylierten Glykolen, ethoxylierten Fettsäuren, nicht-ethoxylierten Fettsäuren, ethoxylierter Saccharose, nicht-ethoxylierter Saccharose, ethoxyliertem Pentaerythrit, nicht-ethoxyliertem Pentaerythrit, ethoxylierten Alkylglycosiden, nicht-ethoxylierten Alkylglycosiden, ethoxylierten Alkylpolyglycosiden. Propylenglykolestern, wie Glycerylstearat, ethoxylierten Sorbitanestern, nicht-ethoxylierten Sorbitanestern, wie Sorbitanstearat, ethoxyliertem Sorbitanstearat, Polysorbat-80, Polysorbat-65, Polysorbat-60, Polysorbat-40, Polysorbat-20, Polyquatermium-37, Glykolstearat, PEG-2-Stearat, PEG-10-Stearat, PEG-30-Stearat, PEG-40-Stearat, PEG-100- Stearat, PEG-150-Stearat, PEG-1000-Stearat, Sorbitanpalmitat, Propylenglykolstearat, Propylenglykol-PEG-25-Stearat, Sorbitanoleat, Metlyglucose-Sesquistearat, Fettalkoholen, wie Cetylalkohol, Stearylalkohol, Cetearylalkohol, propoxylierten und/oder ethoxylierten Fettalkoholen, synthetischen Polymeren, die durch Carboxyvinylpolymere und acoplymere gebildet sind, Acrylaten, Methacrylaten, Alkylacrylaten, Acrylat/ C10-C30-Alkylcrosspolymeren, Acrylamiden, Tauraten, Ethylenoxid- und Propylenoxid-Cpopolymeren sowie aus deren Kombinationen und Derivaten enthält.

8. Kosmetische Zusammensetzung nach Anspruch 1, **dadurch gekennzeichnet, dass** sie ein Sonnenschutzsystem in einer Menge von 0,1 bis 50,0 Gew.-% enthält.

9. Kosmetische Zusammensetzung nach Anspruch 8, **dadurch gekennzeichnet, dass** sie ein Sonnenschutzsystem in einer Menge von 0.1 bis 30,0 Gew.- % enthält.

10. Kosmetische Zusammensetzung nach Anspruch 9, **dadurch gekennzeichnet, dass** das Sonnenschutzsystem aus einer Kombination von Benzylidincampher, Isophthalyliden-campher, Terephthalylidencampher, Zimtsäure, Salicylsäure, Benzoesäure, p-Amino-benzoesäure, substituierten Hydroxybenzophenonen, substituiertem Dibenzoylmethan, Benzotriazolen, wie 2-Arylbenzotriazol, 2-Arylbenzimidazol, 2-Arylbenzolfuranen, 2-Aryl-benzoxazol, 2-Arylindol, mono-Phenylcyanoacrylaten, Diphenylcyanoacrylaten, Triazin-derivaten, wie Hydroxyphenyltriazinverbindungen und Benotriazolderivaten, Amiden, die eine Vinylgruppe enthalten, Zimtsäurederivaten, sulfonierten Benzimidazolen, Diphenylmalonitrilen, Oxalylamiden, Campherderivaten, Salicylsäurederivaten, wie 2-Ethylhexyl-salicylaten, Homosalicylaten und Isopropylsalicylaten, Diphenylacrylaten, Benzophenon-derivaten, wie Benzophenon-2, Benzophenon-3 und Benzophenon-4, PABA, wie 2-Ethyl- hexyl-4-dimethylamino-benzoat, N,N,N-Trimethyl-4-(2-oxoborn-3-yli-denomethyl)-ani- linomethylsulfat, 3,3'-(1,4-Phenylidendimethylen)-bis-(7,7-dimethy-2-oxo-bicyclo-(2,2,1)-1-heptylmethansulfonsäure; 1-(4-Terbutylphenyl)-3-(methoxyphenyl)-propan-1,3-dion; α-(Oxoborn-3-yliden)-toluol-4-sulfonsäure und ihren Kalium-, Natrium- und Triethanol-aminsalzen, 2-Ethylhexyl-2-cyano-3,3'-diphenylacrylat; 2-Ethoxyetyl-4-methoxycinnamat; 2,2'-Dihydroxy-4-methoxybenzophenon; Methylanthanylat; Triethanolaminsalicylat; 2,2',4,4'Tetrahydroxybenzophenon; 2-Phenylbenzimidazol-5-sulfonsäure und ihre Kalium-,Natrium-und Triethanolaminsalze; 2-Ethylhexyl-4-methoxycinnamat; 2-Hydroxy-4-methoxybenzophenon (Oxybenzon); 2-Hydroxy-4-methoxybenzophenonsulfonsäure und ihr Natriumsalz( Sulisobenzon und Natrium-Sulisobenzon); 4-Aminobenzoesäure-PBA-säure; Homomethylsalicylat; N-[(2und4)-([(2-Oxoborn-3-yliden)-methyl]- benzyl]-acrylamid-Polymer;Titandioxid(mit oder ohne lipohilen Überzug); Ethyl-N-ethoxy-4-aminobenzoat; 2-Ethylhexyl-4-dimethylaminobenzoat; 2-Ethylhexylsalicylat; Isobenzyl-4-methoxycinnamat; 3-(4'-Metylbenzyliden)-d-1-campher; 2,4,6-Trianilin-(p-carbo-2-ethylhexyl-1'-oxy)-1,2,5-triazinoctyl; Zinkoxid(mit oder ohne lipophilen Überzug); 2-(2H-Benzotriazol-2-yl)-4-methyl-6-[2-methyl-3-(1,3,3,3-tetramethyl-1-((trimethylsilyl)-oxy)-disiloxanyl)-propyl]-phenol; Benzoesäure; 4,4'-[[6-[[4-[[(1,1-Dimetylethyl]-amino]-carbonyl]-phenyl]-amino]-1,3k,5- triazin-2,4-diyl]-diimino]-bis,bis(2-ethylhexyl): 2,2'-Methylen-bis-6-(2H-benzotriazol-2-yl)-4-(tetramethylbutyl)-1,1,3,3-phenol; Methylen-bis-benzotriazolyl-tetraethylbutylphenol; Mononatriumsalz der 2,2'-bis-(1,4-Phenylen-1H-benzimidazol-4,6-disulfonsäure; (1,3,5)-Triazin-2,4-bis-[[4-(ethylhexyloxy)2-hydroxy]-6-(methoxyphenyl); bis-Ethylhexyloxyphenolmethoxyphenyltriazin; Methylen-bis-benzotriazolyltetramethyl-butylphenol; Butylmethoxydibenzoylmethan; 2-Ethylhexyl-p-methoxycinnamat; Methylen-bis-benzotriazolyltetramethylbutylphenol; 4-Butylmethoxydibenzolmethan; bis-Ethyl-hexyloxyphenolmethoxyphenyltriazin; Octyltriazon: Titandioxid; sowie aus deren Derivaten und Kombinationen zusammengesetzt ist.

11. Kosmetische Zusammensetzung nach Anspruch 10, **dadurch gekennzeichnet, dass** das Sonnenschutzsystem aus Benzophenon-3, Butylmethoxydibenzoylmethan, 2-Ethylhexyl-p-methoxycinnamat und bis-Ethylhexyloxyphenolmethoxytriazin zusammengesetzt ist.

12. Kosmetische Zusammensetzung nach Anspruch10, **dadurch gekennzeichnet, dass** das Sonnenschutzsystem aus Cinnamidopropyltrimoniumchlorid und Dimethylpabamidopropyl-laurdimoniumtosylat zusammengesetzt ist.

13. Kosmetisches Produkt, **dadurch gekennzeichnet, dass** es die kosmetische Zusammensetzung nach einem der Ansprüche 1 bis 12 enthält.

14. Kosmetisches Produkt nach Anspruch 13, **dadurch gekennzeichnet, dass** es einen Körper-Sonnenschutz für die Haut von Erwachsenen und Kindern darstellt.

15. Kosmetisches Produkt nach Anspruch 13, **dadurch gekennzeichnet, dass** es ein Körper- oder ein Gesichts-Befeuchtungsprodukt darstellt.

16. Kosmetisches Produkt nach Anspruch 13, **dadurch gekennzeichnet, dass** es ein Selbst-Bräunungsprodukt darstellt.

17. Kosmetisches Produkt nach Anspruch 13, **dadurch gekennzeichnet, dass** es ein Insekten-Abwehrprodukt darstellt.

18. Kosmetisches Produkt nach Anspruch 13, **dadurch gekennzeichnet, dass** es ein pharmazeutisches Präparat darstellt.

19. Kosmetisches Produkt nach Anspruch 13, **dadurch gekennzeichnet, dass** es ein Sonnen-schutzmittel für Haar darstellt.

20. Kosmetisches Produkt nach Anspruch 13, **dadurch gekennzeichnet, dass** es ein nicht abspülbares Haar-Konditionierungsmittel darstellt.

21. Kosmetisches Produkt nach Anspruch 13, **dadurch gekennzeichnet, dass** es ein abspülbares Haarpräparat darstellt.

22. Verwendung der kosmetischen Zusammensetzung nach einem der Ansprüche 1 bis 12, **dadurch gekennzeichnet, dass** sie zur Herstellung eines kosmetischen Produkts zur Pflege oder zum Schutz der Haut oder des Haares verwendet wird.

23. Verwendung nach Anspruch 22, **dadurch gekennzeichnet, dass** das kosmetische Produkt ein Sonnenschutzmittel für die Haut von Erwachsenen und Kindern darstellt.

24. Verwendung nach Anspruch 22, **dadurch gekennzeichnet, dass** das kosmetische Produkt ein Körper- oder Hautbefeuchtungsprodukt darstellt.

25. Verwendung nach Anspruch 22, **dadurch gekennzeichnet, dass** das kosmetische Produkt ein Selbst-Bräunungsprodukt darstellt.

26. Verwendung nach Anspruch 22, **dadurch gekennzeichnet, dass** das kosmetische Produkt ein Insekten-Abwehrprodukt darstellt.

27. Verwendung nach Anspruch 22, **dadurch gekennzeichnet. dass** das kosmetische Produkt ein pharmazeutisches Präparat darstellt.

28. Verwendung nach Anspruch 22, **dadurch gekennzeichnet, dass** das kosmetische Produkt ein Sonnenschutzmittel für Haar darstellt.

29. Verwendung nach Anspruch 22, **dadurch gekennzeichnet, dass** das kosmetische Produkt ein nicht abspülbares Haar-Konditionierungsmittel darstellt.

30. Verwendung nach Anspruch 22, **dadurch gekennzeichnet, dass** das kosmetische Produkt ein abspülbares Haarpräparat darstellt.

## Revendications

1. Composition cosmétique **caractérisée en ce qu'**elle comprend un système filmogène contenant au moins du cyclopentasiloxane, du diméthiconol, de la diméthicone, du siloxysilicate de triméthyle et de la stéaryl diméthicone et un système émulsifiant contenant au moins de la PEG-8 diméthicone.

2. Composition cosmétique selon la revendication 1, **caractérisée en ce que** la quantité du système filmogène va de 0,3 % à 92,0 % en poids.

3. Composition cosmétique selon la revendication 2, **caractérisée en ce que** la quantité du système filmogène va de 0,5 % à 50,0 % en poids.

4. Composition cosmétique selon la revendication 1, **caractérisée en ce que** la quantité du système émulsifiant va de 0,1 % à 28,0 % en poids.

5. Composition cosmétique selon la revendication 4, **caractérisée en ce que** la quantité du système émulsifiant va de 0,3 % à 12,0 % en poids.

6. Composition cosmétique selon la revendication 1, **caractérisée en ce que** le système filmogène comprend de plus un agent filmogène facultatif choisi dans le groupe constitué par la cyclométhicone, une alkyldiméthicone, un copolyol de diméthicone, la phényl triméthicone, la caprilyl triméthicone, les silicones aminofonctionnelles, les silicones modifiées par un phényle, la phényl triméthicone, les silicones modifiées par un alkyle, un diméthyl et diéthyl polysiloxane, un alkyl en C₁-C₃₀ polysiloxane mixte, le diméthyl siloxane, un o-polyméthyl-siloxane, un α-méthyl-ω-méthoxypolyméthylsiloxane, un polyoxydiméthylsilylène, une huile de polydiméthyle silicone, les élastomères de silicone tels qu'un polymère croisé de cyclométhicone et de diméthicone, un polymère croisé de vinyl diméthicone et de diméthicone, un polymère croisé de diméthicone et de diméthicone et un polymère croisé de cyclopentasiloxane et de diméthicone, les polymères tels qu'une polyvinylpyrrolidone et les alcools polyvinyliques et les combinaisons de ceux-ci.

7. Composition cosmétique selon la revendication 1, **caractérisée en ce que** le système émulsifiant comprend un système émulsifiant supplémentaire choisi dans le groupe constitué par : la lécithine de soja, l'éthoxylate de nonyl phénol, les alkyles phosphates de sodium ou de potassium, le cétyle phosphate de diéthanolamine, les copolymères de diméthicone tels que les polyoxyalkylène diméthyl-polysiloxanes modifiés, le méthyl glucose, le propyl-glycéryle glucose, les glycoles éthoxylés, les glycoles non éthoxylés, les acides gras éthoxylés, les acides gras non éthoxylés, le saccharose éthoxylé, le saccharose non éthoxylé, le pentaérythritol éthoxylé, le pentaérythritol non éthoxylé, les alkyles glycosides éthoxylés, les alkyles glycosides non éthoxylés, les alkyles polyglycosides éthoxylés, le propylèneglycol, les esters tels que le stéarate de glycéryle, les esters de sorbitane éthoxylés, les esters de sorbitane non éthoxylés tels que le stéarate de sorbitane, le stéarate de sorbitane éthoxylé, le polysorbate 80, le polysorbate 65, le polysorbate 60, le polysorbate 40, le polysorbate 20, le polyquaternium 37, le stéarate de glycol, le stéarate de PEG-2, le stéarate de PEG-10, le stéarate de PEG-30, le stéarate de PEG-40, le stéarate de PEG-100, le stéarate de PEG-150, le stéarate de PEG-1000, le palmitate de sorbitane, le stéarate de propylèneglycol, le stéarate de propylèneglycol et de PEG-25, l'oléate de sorbitane, le sesquistéarate de méthyl glucose, les alcools gras tels que l'alcool cétylique, l'alcool stéarylique, l'alcool cétéarylique, les alcools gras propoxylés et/ou éthoxylés, les polymères synthétiques formés par des polymères et copolymères carboxyvinyliques, les acrylates, les méthacrylates, les acrylates d'alkyle, un polymère croisé acrylates/alkyle en C₁₀-C₃₀, les acrylamides, les taurates, les copolymères d'oxyde d'éthylène et d'oxyde de propylène, et les combinaisons de ceux-ci et les dérivés de ceux-ci.

8. Composition cosmétique selon la revendication 1, **caractérisée en ce qu'**elle comprend un système écran solaire en une quantité allant de 0,1 % à 50,0 % en poids.

9. Composition cosmétique selon la revendication 8, **caractérisée en ce qu'**elle comprend un système écran solaire en une quantité allant de 0,1 % à 30,0 % en poids.

10. Composition cosmétique selon la revendication 9, **caractérisée en ce que** le système écran solaire se compose d'une combinaison entre : le benzylidène camphre, l'isophtalylidène camphre, le téréphthalylidène camphre, l'acide cinnamique, l'acide salicylique, l'acide benzoïque, l'acide p-aminobenzoïque, les hydroxybenzophénones substituées, le dibenzoylméthane substitué, un benzotriazole tel que le 2-arylbenzotriazole, le 2-arylbenzimidazole, les 2-arylbenzofuranes, le 2-arylbenzoxazole, le 2-arylindole, les mono-phénylcyano-acrylates, les diphénylcyanoacrylates, les dérivés de triazine tels que les composés d'hydroxyphényltriazine et les dérivés de benzotriazole, les amides contenant un groupe vinyle, les dérivés de l'acide cinnamique, les benzimidazoles sulfonés, les diphénylmalonitriles, les oxalylamides, les dérivés de camphre, les dérivés de l'acide salicylique tels que les salicylates de 2-éthylhexyle, les homosalates et les salicylates d'isopropyle, les acrylates de diphényle, les dérivés de benzophénone tels que la benzophénone-2, la benzophénone-3 et la benzophénone-4, un PABA tel que le 4-diméthylaminobenzoate de 2-éthylhexyle, le sulfate de N,N,N-triméthyl-4-(2-oxoborn-3-ylidèneométhyl) anilinométhyle, l'acide 3,3'-(1,4-phénylènediméthylène) bis(7,7-diméthyl-2-oxo-bicyclo-(2.2.1) 1-heptylméthanosulfonique ; la 1-(4-ter-butyl-phényl)-3-(4-méthoxyphényl)propane-1,3-dione ; l'acide α-(2-oxoborn-3-ylidène) toluène-4-sulfonique et ses sels de potassium, de sodium et de triéthanolamine ; le 2-cyano-3,3'-diphénylacrylate de 2-éthylhexyle ; le 4-méthoxycinnamate de 2-éthoxyéthyle ; la 2,2'-dihydroxy-4-méthoxy-benzophénone ; l'anthanilate de méthyle ; le salicylate de triéthanolamine ; la 2,2',4,4'-tétrahydroxybenzophénone ; l'acide 2-phénylbenzimidazol-5-sulfonique et ses sels de potassium, de sodium et de triéthanolamine ; le 4-méthoxycinnamate de 2-éthylhexyle ; la 2-hydroxy-4-méthoxy-benzophénone (oxybenzone) ; l'acide 2-hydroxy-4-méthoxy-benzophénone sulfonique et ses sels de sodium (sulisobenzone et sulisobenzone sodique) ; l'acide 4-aminobenzoïque PABA ; le salicylate d'homométhyle ; un polymère de N-{(2 et 4)[(2-oxoborn-3-ylidène)méthyl]benzyl}-acrylamide ; le dioxyde de titane (avec ou sans revêtement lipophile) ; le N-éthoxy-4-aminobenzoate d'éthyle ; le 4-diméthylaminobenzoate de 2-éthylhexyle ; le salicylate de 2-éthylhexyle ; le 4-méthoxycinnamate d'isopentyle; le 3-(4'-méthylbenzylidène-d-1-camphre ; le 3-benzylidène camphre ; le 2,4,6-trianiline-(p-carbo-2-éthyl-hexyl-1'-oxy)-1,2,5-triazine octyle ; l'oxyde de zinc (avec ou sans revêtement lipophile) ; le 2-(2H-benzotriazol-2-yl)-4-méthyl-6-{2-méthyl-3-(1,3,3,3-tétraméthyl-1-((triméthylsilyl)oxy)disiloxanyl)propyl}phénol ; l'acide benzoïque ; le 4,4'-[[6-[[4-[[(1,1-diméthyl-éthyl]amino]carbonyl]-phényl]amino]-1,3k,5-triazine-2,4-diyl]diimino]bis,bis(2-éthylhexyle) ; le 2,2'-méthylène-bis-6-(2H-benzotriazol-2-yl)-4-(tétraméthylbutyl)-1,1,3,3-phénol ; le méthylène bis-benzotriazolyl tétraéthyl butyl phénol ; le sel monosodique de l'acide 2,2'-bis(1,4-phénylène)-1H-benzimidazol-4,6-disulfonique ; le (1,3,5)-triazine-2,4-bis{[4-(2-éthyl-hexyloxy)-2-hydroxy]-6-(4-méthoxyphényl) ; la bis-éthylhexyloxyphénol méthoxyphényl triazine ; le méthylène bis-benzotriazolyl tétraméthylbutylphénol ; le butyl méthoxydibenzoylméthane, le p-méthoxycinnamate de 2-éthylhexyle, le méthylène bis-benzotriazolyl tétraméthylbutylphénol, le 4-butylméthoxydibenzolméthane, la bis-éthylhexyloxyphénol méthoxyphényl triazine, l'octyl triazone, le dioxyde de titane et les dérivés de ceux-ci et les combinaisons de ceux-ci.

11. Composition cosmétique selon la revendication 10, **caractérisée en ce que** le système écran solaire est composé de benzophénone-3, de butyl méthoxydibenzoylméthane, de p-méthoxycinnamate de 2-éthylhexyle et de bis-éthylhexyloxyphénol méthoxyphényl triazine.

12. Composition cosmétique selon la revendication 10, **caractérisée en ce que** le système écran solaire se compose de chlorure de cinnamidopropyltrimonium et de tosylate de diméthylpabamidopropyl laurdimonium.

13. Produit cosmétique **caractérisé en ce qu'**il comprend la composition cosmétique selon l'une quelconque des revendications 1 à 12.

14. Produit cosmétique selon la revendication 13, **caractérisé en ce qu'**il s'agit d'un écran solaire corporel pour la peau des adultes et des enfants.

15. Produit cosmétique selon la revendication 13, **caractérisé en ce qu'**il s'agit d'un produit hydratant pour le corps ou le visage.

16. Produit cosmétique selon la revendication 13, **caractérisé en ce qu'**il s'agit d'un produit auto-bronzant.

17. Produit cosmétique selon la revendication 13, **caractérisé en ce qu'**il s'agit d'un produit insectifuge.

18. Produit cosmétique selon la revendication 13, **caractérisé en ce qu'**il s'agit d'une préparation pharmaceutique.

19. Produit cosmétique selon la revendication 13, **caractérisé en ce qu'**il s'agit d'un écran solaire pour les cheveux.

20. Produit cosmétique selon la revendication 13, **caractérisé en ce qu'**il s'agit d'un après-shampoing sans rinçage.

21. Produit cosmétique selon la revendication 13, **caractérisé en ce qu'**il s'agit d'une préparation capillaire à rincer.

22. Utilisation d'une composition cosmétique selon les revendications 1 à 12, **caractérisée en ce qu'**elle se rapporte à la production d'un produit cosmétique pour le soin et la protection de la peau ou des cheveux.

23. Utilisation selon la revendication 22, **caractérisée par le fait que** le produit cosmétique est un écran solaire pour la peau des adultes et des enfants.

24. Utilisation selon la revendication 22, **caractérisée par le fait que** le produit cosmétique est un produit hydratant pour le corps ou le visage.

25. Utilisation selon la revendication 22, **caractérisée par le fait que** le produit cosmétique est un produit auto-bronzant.

26. Utilisation selon la revendication 22, **caractérisée par le fait que** le produit cosmétique est un produit insectifuge.

27. Utilisation selon la revendication 22, **caractérisée par le fait que** le produit cosmétique est une préparation pharmaceutique.

28. Utilisation selon la revendication 22, **caractérisée par le fait que** le produit cosmétique est un écran solaire pour les cheveux.

29. Utilisation selon la revendication 22, **caractérisée par le fait que** le produit cosmétique est un après-shampoing sans rinçage.

30. Utilisation selon la revendication 22, **caractérisée par le fait que** le produit cosmétique est une préparation capillaire à rincer.
